(19) Europäisches Patentamt
European Patent Office
Office européen des brevets

(11) Publication number: 0 527 661 A1

# (12) EUROPEAN PATENT APPLICATION

(21) Application number: **92307490.0**

(22) Date of filing: **14.08.92**

(51) Int. Cl.5: **C07F 3/00**, C07F 5/00, C07C 49/92, C07C 45/77, C23C 16/00, //C07C49/167

(30) Priority: **14.08.91 GB 9117562**

(43) Date of publication of application: **17.02.93 Bulletin 93/07**

(84) Designated Contracting States: **AT BE CH DE DK ES FR GB GR IE IT LI LU NL PT SE**

(71) Applicant: **THE ASSOCIATED OCTEL COMPANY LIMITED**
**20 Berkeley Square**
**London W1X 6DT(GB)**

(72) Inventor: **Cole-Hamilton, David John**
**St Rule**
**By St Andrews, Fife KY16 9ST(GB)**
Inventor: **Thompson, Simon Carl**
**40 Haxby Road**
**York(GB)**
Inventor: **Cook, Stephen Leonard**
**3 Buckingam Avenue**
**Chester(GB)**
Inventor: **Barr, Donald**
**5 Dunmore Road**
**Little Sutton, South Wirral L66 4PD(GB)**

(74) Representative: **Lambert, Hugh Richmond et al**
**D. YOUNG & CO. 10 Staple Inn**
**London, WC1V 7RD (GB)**

## (54) Metal beta-diketonates.

(57) Novel metal β-diketonates are disclosed, including coordination complexes thereof with inorganic and organic donor ligands, useful in CVD applications. These are metal β-diketonates of the formula:

$M\{R^1C(O)CHC(O)R^2\}_x$

where

M is a metal cation of valency x, preferably a cation of an alkaline earth metal or rare earth metal;

$R^1$ is $C_1$-$C_8$ perfluoroalkyl, preferably $C_1$-$C_5$ perfluoroalkyl, preferably straight chain e.g. $-CF_3$ or n-$C_3F_7$;

$R^2$ is $C_2$-$C_8$ perfluoroalkyl, preferably $C_2$-$C_6$ perfluoroalkyl, again preferably straight chain e.g. n-$C_3F_7$ or n-$C_6F_{13}$;

and their donor ligand complexes, with either an inorganic donor ligand such as $H_2O$, or an organic donor ligand (Lewis base), or both. Also disclosed is a novel route for the preparation of such complexes.



Rank Xerox (UK) Business Services
(3.10/3.5x/3.0.1)

## FIELD OF INVENTION

This invention relates to novel metal β-diketonates of potential utility in chemical vapour deposition (CVD) processes and in other chemical processes where the volatility of the reagents or their solubility in organic solvents is desirable, a process for the preparation thereof, and their use in CVD processes.

## BACKGROUND INFORMATION AND PRIOR ART

The chemical vapour deposition (CVD) of thin metal containing, e.g. oxide or fluoride, films is of considerable interest and of potential utility in the production of thin films of high temperature superconductors. Of particular interest in this field are thin oxide or fluoride films comprising one or more alkaline earth metals or rare earth metals and mixtures thereof, in addition to copper and main group elements such as Tl, Pb or Bi.

Volatile alkaline earth metal compounds are known, including, for example, the β-diketonates of calcium, barium and strontium, see Anal. Chem., 1970, 42, 1828 and "Comprehensive Coordination Chemistry", Ed. G. Wilkinson, Vol. 3, Chapter 23.4.1., page 25 et. seq.

These β-diketonates of Gp.II metals are, however, alleged to be thermally unstable and therefore of reduced utility for CVD processes.

In EP-A-0405634 volatile Gp.II metal β-diketonate complexes are disclosed which are alleged to be of improved volatility and thermal stability, this being achieved by complexing the β-diketonate with either a neutral oxygen donor ligand e.g. a cyclic or acyclic ether such as $(CH_2CH_2O)_6$, a glycol ether such as $R-(CH_2CH_2O)_nCH_3$, where R = OH or $OCH_3$ and n is 1 to 6, an alcohol, a ketone, an aldehyde, an anhydride or amide, or in the case of barium and strontium compounds a neutral nitrogen donor ligand such as a nitrile or dinitrile, a mono-, di- or polyamine, a pyridine or dipyridine, phenanthroline or an imine or diimine. Such complexes are represented by the formula

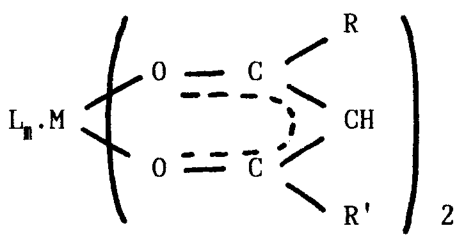

where

L = the ligand molecule;

m = the number of ligand molecules;

M = Ba, Sr or Ca;

R and R' = alkyl or aryl, substituted alkyl or aryl and especially fluoro-substituted alkyl or aryl.

Especially preferred are the complexes of Gp.II β-diketonates where, in the formula of the β-diketonate at least one of the groups is either $-CF_3$ or $n-C_3F_7$. The specific examples given are all of Gp.II metal β-diketonates derived from the β-diketones:

$CF_3C(O)CH_2C(O)CF_3$,

$CF_3C(O)CH_2C(O)CH_3$,

and $n-C_3F_7C(O)CH_2C(O)C(CH_3)_3$

Typical examples are the complexes:

Ba $\{n-C_3F_7C(O)CHC(O)C(CH_3)_3\}_2.\{CH_3O(CH_2CH_2O)_6CH_3\}$

Ba $\{CF_3C(O)CHC(O)CF_3\}_2.2\{CH_3O(CH_2CH_2O)_2CH_3\}$

Ba $\{CF_3C(O)CHC(O)CH_3\}_2.\{(CH_2CH_2O)_6\}$

Sr $\{CF_3C(O)CHC(O)CF_3\}_2.2\{CH_3OCH_2CH_2OCH_3\}$

and Ca $\{CF_3C(O)CHC(O)CF_3\}_2.2\{CH_3OCH_2CH_2OCH_3\}$

Such complexes are prepared by reacting the Gp.II metal β-diketonate e.g.

Ba $\{CF_3C(O)CHC(O)CF_3\}_2$

or Ba $\{CF_3C(O)CHC(O)CH_3\}_2$

or Ba $\{n-C_3F_7C(O)CHC(O)C(CH_3)_3\}_2$

or the corresponding Ca or Sr compound, in suspension in toluene with the ligand followed by evaporating

to dryness. The complexes thus prepared are shown to sublime when heated in vacuo (0.01 to 5mm Hg) to temperatures in the range 85 to 180°C.

CVD of metal fluorides is of particular importance in the preparation of high-purity glasses and of the deposition of electrically insulating layers in semiconductor technology, and as high temperature lubricants.

To be completely suitable as a precursor for CVD purposes, amongst other properties, any complex should exhibit an indefinite thermal stability at the temperatures at which it will be used. Temperatures of use will be those temperatures at which, under technically feasible conditions of low pressures and inert gas flow rates, industrially acceptable carry-over and deposition rates are obtained. In general, for CVD of high Tc superconducting thin films, precursors are likely to be used at temperatures of the order of 100 to 200°C, pressures of about 1 to 20 mBar, and inert gas flow rates of a few tens to a few hundreds of standard centimetres cubed per minute (sccm).

The purpose of the above is such that once a precursor has been charged to a deposition apparatus, repeated depositions can be obtained from the one sample. Further, it is important, for multi-component films, that conditions required (temperature, pressure, flow rate) to give certain, predictable mass flows (carry-over) of precursor do not change with time. Deposition trials should show reliable and reproducible carry-over and deposition rates for a number of runs, preferably over an extended period of time.

Likely stability of a precursor under in-use conditions may be inferred from Simultaneous Thermal Analysis (STA) data and sublimation studies. A material exhibiting evaporation to zero or near zero residue at ambient pressure probably has acceptable volatility and certainly acceptable thermal stability. Complete evaporation does not in itself show that the composition of a precursor does not change with time under conditions of use. For example, decomposition may occur to leave a volatile residue. This would normally be expected to give problems with reproducibility of carry-over rate. This is so in the case of Lewis base complexes of the barium bis($\beta$-diketonates) disclosed in EP-A-0405634, particularly where the diketonate is heavily fluorinated. Loss of the Lewis base can leave a volatile material. Recovery of unchanged material, with good mass balance, from vacuum sublimation at temperatures and pressures approximating to those of use provides very good evidence that such decomposition does not occur.

Under the conditions of temperature, pressure and insert gas flow rate frequently required for CVD of high Tc superconducting oxides, equilibrium vapour pressures of the precursor are rarely attained within the precursor pot. This often means that the surface area:volume ratio of the precursor is important in determining the carry-over rate of material. For reproducible CVD it is important that carry-over rate does not change with time, assuming otherwise constant conditions. This means that the precursor should preferably be liquid, or if solid, not be prone to sintering at the temperature of use. Sintering is not usually encountered if the melting point is at least 10°C, and preferably 20°C, above the operating temperature. Overall, this means that typically, for a barium precursor, the melting point should be below about 130°C or above about 190°C.

The fluorinated $\beta$-diketones of the alkaline earth metals and their Lewis base coordinated analogues are well known in the art to deposit fluoride salts rather than oxides. This is the basis of their application as electrically insulating layers. It is also well known that deposition of the oxide can be achieved by ensuring that a small partial pressure of water is present during deposition. This is described in, for example, Malanerino et al. Appl. Phys. Lett., 1991, 58, 182-184, and Wills et al, Ibid., 1992, 60, 41-43.

Additional prior art in the field of alkali, alkaline earth and other metal $\beta$-diketonates for use in CVD applications includes US Patent No. 4558144, which discloses adducts of magnesium and zinc $\beta$-diketonates with the bidentate ligand 1,2-dimethoxyethane, and more especially such adducts prepared from hexafluoroacetylacetone as the $\beta$-diketone, i.e. the compound:

$CF_3C(O)CH_2C(O)CF_3$.

Crown ligand complexes of the hexafluoroacetylacetonate salts of alkaline earth metals and lanthanide metals are disclosed for CVD applications in EP-A-0460627.

Coordination complexes of thallium, lead and calcium hexafluoroacetylacetonates and nitrogen-containing organic donor ligands (Lewis base) are disclosed in J. Chem. Soc. Dalton Trans., 1974, 655-657.

The above prior art is merely representative of the extensive prior art relating to CVD processes using metal $\beta$-diketonates as the volatile metal source, and is not intended in any way to be exhaustive.

## OBJECTS OF THE INVENTION

The object of the present invention is to provide new and improved metal $\beta$-diketonates for CVD processes, in particular, metal, and especially alkaline earth metal $\beta$-diketonates, which are of improved

stability and volatility in comparison with other β-diketonates already known in the art, and including their donor ligand complexes.

A further object is to provide improved CVD methods using such novel and improved β-diketonates, and including the donor ligand complexes thereof.

A further object is to provide improved methods for the preparation of such donor ligand complexes of β-diketonates.

## SUMMARY OF THE INVENTION

In accordance with the present invention, it has now been found that metal β-diketonates, and especially the Gp.II and rare earth metal β-diketonates, of the β-diketones
$CF_3C(O)CH_2C(O)n\text{-}C_3F_7$
Decafluoro-3H-hepta-2,4-dione (DFHD)
$n\text{-}C_3F_7C(O)CH_2C(O)n\text{-}C_3F_7$
Tetradecafluoro-5H-nona-4,6-dione (TDFND)
$n\text{-}CF_3C(O)CH_2C(O)n\text{-}C_6F_{13}$
Hexadecafluoro-3H-deca-2,4-dione (HDFDD)
$n\text{-}C_3F_7C(O)CH_2C(O)n\text{-}C_6F_{13}$
Eicosafluoro-5H-dodeca-4,6-dione (EF3D)
and similar compounds, especially those based on TDFND, show unexpected volatility and thermal stability, even in the absence of a neutral oxygen or nitrogen donor ligand, and/or other desirable properties. Such compounds are therefore of considerable value as volatile metal compounds in CVD applications, and in many other applications. Such compounds are believed to be novel, but with one possible exception. In the on-line Chemical Abstracts Database "CAS-ON-LINE" there is a listing of the compound

$Ba(\underline{n}\text{-}C_3F_7\text{-}C(O)\text{-}CH\text{-}C(O)\text{-}\underline{n}\text{-}C_3F_7)_2$

under Registry No. 134316-31-7. No preparative details are given, nor any source, either for that compound or the abstract, nor any further information of any kind concerning that compound, i.e. whether it was or ever has been prepared or how.

## BRIEF DESCRIPTION OF THE DRAWINGS

Thermal analysis and other data relating to the compounds of the present invention and their utility in CVD applications is presented in the accompanying drawings, in which:

Figure 1 is the Simultaneous Thermal Analysis (STA) trace of the compound $Ca(DFHD)_2.H_2O$. The STA trace, in fact, consists of two separate traces: the Thermo-Gravimetric Analysis (TGA) trace plotting percentage weight loss against temperature, and the Differential Thermal Analysis (DTA) trace recording the difference in temperature between the measured temperature of the sample and the measured temperature of standard, thermally stable, solid, non-volatile material being heated at precisely the same rate. Peaks in the DTA trace therefore represent exotherms experienced during heating of the sample and due for example to decomposition, whilst the troughs represent endothermic events, e.g. melting and volatilisation. Comparison of the two traces enables a profile to be built up of the thermal stability of the present compounds.

Figure 2 is the STA trace for the compound $Ca(TDFND)_2.H_2O$.
Figure 3 is the STA trace for the compound $Sr(DFHD)_2.H_2O$.
Figure 4 is the STA trace for the compound $Sr(TDFND)_2.H_2O$.
Figure 5 is the STA trace for the compound $Ba(DFHD)_2.H_2O$.
Figure 6 is the STA trace for the compound $Ba(TDFND)_2.H_2O$.
Figure 7 is the STA trace for the compound (anhyd.)$Ba(TDFND)_2$.
Figure 8 is the STA trace for the compound (anhyd.)$Ba(EF3D)_2$.
Figure 9 is the STA trace for the compound (anhyd.)$Ba(HDFDD)_2$.
Figure 10 is the STA trace for the complex $Ba(TDFND)_2$.tetraglyme.
Figure 11 is the STA trace for the complex $Ba(TDFND)_2$.18-crown-6.
Figure 12 is the STA trace for the complex $Ba(TDFND)_2$.2(diglyme).
Figure 13 is the STA trace for the complex $Ba(EF3D)_2$.18-crown-6.
Figure 14 is the STA trace for the complex $Ba(HDFDD)_2$.18-crown-6.
Figure 15 is the STA trace for the complex $Ba(EF3D)_2$.tetraglyme.

Figure 16 is the STA trace for the complex $Ba(HDFDD)_2$.tetraglyme.
Figure 17 is an Arrhenius plot of $BaF_2$ deposition from $Ba(TDFND)_2$ over the temperature range 623 to 1023K.
Figure 18 is an Arrhenius plot of $BaF_2$ deposition from $Ba(TDFND)_2$ over the temperature range 623 to 673K.

## DETAILED DESCRIPTION

In accordance with the present invention, therefore, there are provided, as novel compounds, metal β-diketonates of the formula I:

$$M\ \{R^1C(O)CHC(O)R^2\}_x \qquad I$$

where
M is a metal atom of valency x, preferably an alkaline earth or rare earth metal;
$R^1$ is $C_1$-$C_8$ perfluoroalkyl, preferably $C_1$-$C_5$ perfluoroalkyl, preferably straight chain e.g. $-CF_3$, $-C_2F_5$, or n-$C_3F_7$;
and $R^2$ is $C_2$-$C_8$ perfluoroalkyl, preferably $C_3$-$C_6$ perfluoroalkyl, again preferably straight chain e.g. n-$C_3F_7$, or n-$C_6F_{13}$;
most preferably $R^1 = R^2 =$ n-$C_3F_7$.

In a second aspect of the invention, there is provided a method of forming metal containing films, and especially alkali metal and alkaline earth metal containing films, including mixed metal containing films, on a substrate, which comprises contacting the substrate under chemical vapour deposition conditions with volatile compounds of one or more metals in vapour phase, thereby to deposit on the surface of the substrate a film containing the metal or metals in question, which may be an oxide film or a fluoride film, depending on the composition of the vapour used in the CVD process, more especially the water content, wherein the said volatile metal compound is or comprises a metal β-diketonate of formula I above. Preferred substrate materials include glass, metal, semiconductor material, such as silicon, ceramics, and metal oxides such as MgO (sapphire), yttrium stabilised zirconia, strontium titanate, etc.

The metal β-diketonates of the present invention are readily prepared by the reaction of a metal oxide, hydroxide, halide or other salt in aqueous solution with the appropriate diketone, in the presence of a base, e.g. an aqueous alkali metal hydroxide. Preferably the reaction is performed in an aqueous alcoholic solution.

The preparation of the compounds of this invention is illustrated by the following Examples.

## EXAMPLE 1

### Preparation of the β-diketones DFHD(1), TDFND(2), HDFDD(3) and EF3D(4)

Sodium methoxide (7.8g, 0. 145mol) was suspended in dry petrol ($75cm^3$) with vigorous stirring. Ethylheptafluorobutyrate (32.3g, 0.133mol) was added to the flask from a dropping funnel over 30 minutes. After a further 30 minutes stirring trifluoroacetone (17.2g, 0.153mol) was added dropwise over one hour. The solution was left to stand overnight. The solution was then acidified with $H_2SO_4$ ($50cm^3$, $6mol\ dm^{-3}$) with vigorous stirring in an ice bath. The two phases were separated in a separating funnel. The aqueous phase was extracted three times with petrol ($20cm^3$) and added to the original petrol phase. This was then extracted with aqueous sodium acetate ($20cm^3$, $260g\ dm^3$). This aqueous phase was added to a stirred solution of $CuSO_4 5H_2O$ (12.6g) in water ($75cm^3$). The resultant green precipitate was filtered, washed sparingly with iced water then vacuum dried. This solid, identifiable as $Cu(DFHD)_2$, was then placed in an ice bath and cold, concentrated sulphuric acid ($3x5cm^3$) was added. A green liquid above a white suspension was produced. This liquid was filtered off and distilled using a Vigreux flask, the product cut, DFHD(1), came over at 100 to 104 °C.

In a second experiment the above procedure was repeated using methylheptafluoropropylketone in place of trifluoroacetone. The product fraction TDFND(2) was collected at 134 to 135 °C.

The $^1H$ nmr of both compounds gives the expected spectrum for the enol form with peaks at δ6.6 and δ12.9 in a 1:1 ratio.

13C nmr for TDFND(2)

$CDCl_3$ solution, shifts in ppm relative to TMS, δ114.5 (q of t, $J_{C\text{-}F}$ 287 Hz, $J_{C\text{-}F}$ 33 Hz, $CF_3$), δ115.11 (m, $CF_3CF_2$), δ109.24 (t of t, $J_{C\text{-}F}$ 33 Hz, $CF_2CO$), δ178.86 (t, $J_{C\text{-}F}$ 29 Hz, Co), δ95.41 (s, $CH_2$).

In a third experiment, the above procedure was repeated using trifluoroacetone as the ketone and ethylperfluoroheptanoate as the ester. On extraction of the petroleum solution with aqueous sodium acetate, most of the β-diketone was found to separate as an oil. The product HDFDD (hexadecafluorodecanedione)-(3) recovered by cautious acidification of the copper complex was identified by GCMS and found to be sufficiently pure for use in preparation of the barium complexes.

In a fourth experiment, the procedure was repeated using methylheptafluoropropylketone and ethylperfluoroheptanoate as with HDFDD above, to yield acceptably pure EF3D (eicosafluorododecanedione)(4), also identified by GCMS.

## EXAMPLE 2

### Preparation of Barium $(TDFND)_2$

Tetradecafluorononanedione (4.08g, 0.01mol) TDFND prepared as in Example 1 was dissolved in 50% aqueous ethanol ($20cm^3$). To this was slowly added sodium hydroxide (0.40g, 0.01mol) dissolved in 50% aqueous ethanol ($20cm^3$).

This was then slowly added to barium bromide (1.67g, 0.005mol) dissolved in 50% aqueous ethanol ($30cm^3$).

The volume of the solution was then reduced by c.25% under vacuum and water ($50cm^3$) was added. The solution was again put under vacuum until the product precipitated. The solid was retrieved by filtration and dried under vacuum.

The product $^1H$ nmr displays only one peak which is attributable to the product, a singlet at δ5.9. This is clearly the hydrogen on carbon 5. The proton from the OH group has, as expected, disappeared. Resonance attributable to the presence of one molecule of water is apparent, and accordingly the product is assigned the formula $Ba(TDFND)_2.H_2O$.

Microanalysis data is as follows:
Found C 22.17%, H 0.35% (error ± 0.3%)
$C_{18}H_4BaF_{28}O_5$ requires C 22.23%, H 0.41%

Following recovery the hydrated product is heated in vacuo at 100°C to yield the anhydrous product $Ba(TDFND)_2$.

Product analysis:
Found C 22.62%, H 0.13% (error ± 0.3%)
$C_{18}H_2BaF_{28}O_4$ requires C 22.71%, H 0.21%

## EXAMPLE 3

Following the same general procedure of Example 2, the Gp.II metal β-diketonates shown in the following table have been prepared and tested:

TABLE OF VOLATILITIES AND MOLECULAR FORMULAE OF DIKETONATES

| DIKETONATE | TEMP. OF SUBLIMATION (°C)$^+$ (40 Torr) | PROBABLE MOLECULAR FORMULA |
|---|---|---|
| $Ba(TDFND)_2$ | 190 | $Ba(TDFND)_2.H_2O$ |
| $Sr(TDFND)_2$ | (1) | $Sr(TDFND)_2.H_2O$ |
| $Ca(TDFND)_2$ | <160 | $Ca(TDFND)_2.H_2O$ |
| $Ba(DFHD)_2$ | 240 | $Ba(DFHD)_2.H_2O$ |
| $Sr(DFHD)_2$ | 195 | $Sr(DFHD)_2.H_2O$ |
| $Ca(DFHD)_2$ | 170 | $Ca(DFHD)_2.H_2O$ |
| $Y(DFHD)_3$ | 165 | $Y(DFHD)_3.H_2O$ |
| $Y(TDFND)_3$ | 140 | $Y(TDFND)_3.H_2O$ |

Notes:
(1) Sublimes directly to collection flask, difficult to determine sublimation point.

EXAMPLE 4

Preparation of Barium Bis(β-diketonates) Under Anhydrous Conditions

A Schlenk tube was charged in a nitrogen-filled dry box with barium hydride ($BaH_2$, 0.695g, 5mmol). The tube was sealed, removed from the box and attached to the nitrogen manifold of a Schlenk line. The tube was then charged with dry toluene (10cm$^3$) and diethyl ether (10cm$^3$), by syringe, against a strong flow of nitrogen. Finally, TDFND (2.5 cm$^3$, 10mmol) was added, dropwise, with caution, also against nitrogen flow. After a strong initial effervescence had died down, some white solids formed at the liquid surface. These were dissolved by the addition of further diethylether (5cm$^3$) accompanied by gentle warming. After about thirty minutes the resulting clear, yellow-tinged solution was filtered and evaporated to dryness under vacuum. A white emulsion formed, from which an oily solid precipitated. On further drying, a pale yellow powder formed. This was pumped on exhaustively. An essentially quantitative yield of $Ba(TDFND)_2$ was isolated.

The melting point of this material was identical to that of material formed in Example 2, following dehydration. Satisfactory C, H analysis was obtained.

EXAMPLE 5

In a further experiment, $BaH_2$ was reacted with EF3D using the procedure of Example 4. Isolation of solids required several cycles of freezing with liquid nitrogen, followed by melting, both under vacuum. A greyish powder was recovered in 97% yield.

EXAMPLE 6

In a further experiment, HDFDD was used as the β-diketonate in the procedure of Example 4. A golden yellow, glassy solid was recovered on evaporation to dryness under vacuum. The yield was, again, 97%.

Thermal data on the compounds prepared is shown in the accompanying drawings which show the Simultaneous Thermal Analysis (STA) traces of the compounds:

| | |
|---|---|
| $Ca(DFHD)_2.H_2O$ | Figure 1 |
| $Ca(TDFND)_2.H_2O$ | Figure 2 |
| $Sr(DFHD)_2.H_2O$ | Figure 3 |
| $Sr(TDFND)_2.H_2O$ | Figure 4 |
| $Ba(DFHD)_2.H_2O$ | Figure 5 |
| $Ba(TDFND)_2.H_2O$ | Figure 6 |
| $Ba(TDFND)_2$.anhydrous (Example 4) | Figure 7 |
| $Ba(EF3D)_2$.anhydrous (Example 5) | Figure 8 |
| $Ba(HDFDD)_2$.anhydrous (Example 6) | Figure 9 |

In these traces the first trace (percent scale) is the Thermo-Gravimetric Analysis (TGA) trace, whilst the second trace (microvolt scale) is the Differential Thermal Analysis (DTA) trace, i.e. recording the difference in temperature between the measured temperature of the sample, and the measured temperature of a standard (non-volatile, non-decomposable) material experiencing the same rate of heating. Upward peaks on the DTA trace therefore represent exothermic events, whilst downward peaks represent endothermic events. The TGA trace simply shows weight loss with increasing temperature and which may be due either to decomposition or volatilisation. However, since the products of decomposition will be largely non-volatile, weight loss due to volatilisation can be distinguished from weight loss due to decomposition by reference to the amount of residue remaining and as shown on the TGA trace, and by reference to the DTA trace where volatilisation appears as a endothermic event, and thus appearing on the trace as a downward peak coincident with the weight loss, whereas weight loss due to decomposition appears usually as a exothermic (but occasionally endothermic) event.

The STA measurements were conducted at atmospheric pressure under an atmosphere of flowing nitrogen.

Referring to the accompanying drawings the STA traces for $Ca(DFHD)_2.H_2O$ and $Ca(TDFND)_2.H_2O$, Figures 1 and 2, show rapid weight loss occurring at about 200 °C accompanied by a small endotherm, and a relatively small constant weight residue, indicating some decomposition.

A similar pattern is to be seen for the two strontium compounds (Figures 3 and 4). Here the endothermic (downward) peaks occurring at about 67°C (Figure 3) and 54°C (Figure 4) are accompanied by a small weight loss and attributable to the loss of water from the complex. The endothermic peaks at around 155°C (Figure 3) and 100°C (Figure 4) are not accompanied by any weight loss and are attributable to melting.

In Figure 5, $Ba(DFHD)_2.H_2O$, the DTA trace shows clear endothermic events with onsets at about 83°C and 197°C. A more gradual event begins at about 250°C, and an exothermic peak at 330°C. Examination of the TGA trace allows these events to be explained as follows. The small weight loss between 100 and 120°C is associated with the first endothermic peak and is due to driving off water ligand. The 197°C event has no weight loss and is a melt. The gentle endothermic event is associated with the major weight loss. This is probably due to volatilisation, but the sizeable residue and the possibly exothermic event at 330°C suggest that this is accompanied by some decomposition.

In Figure 6, $Ba(TDFND)_2.H_2O$, the TGA trace shows a very small weight loss at about 70 to 120°C. There is then a gradual onset of a weight loss to a small residue. These observations correspond to events in the DTA trace. The hump between 60 and 120°C is due to the driving off of bound water. The event starting at 188°C is then a melt, which is followed by a rising endotherm, which cuts off at about 300°C, at which point the rate of weight loss falls to zero.

The STA traces (Figure 7) for the anhydrous compound $Ba(TDFND)_2$ show no endotherm in the region 100°C, indicating absence of bound water. Within the limits of experimental error the m.p. of the anhydrous complex is identical to that of the anhydrous compound obtained by dehydration of the hydrated complex (Example 2), viz. m.p. onset circa 188 to 190°C. As shown in Figure 7, an endothermic event follows, accompanied by a weight loss. The low residue remaining after that event indicates volatilisation of the compound with minimal decomposition.

The compound $Ba(EF3D)_2$, Example 5, shows a broad melting point range onsetting at about 80°C (Figure 8). In the TGA tram a small weight loss with an associated endotherm is seen over the range 130 to 180°C, believed, in this case, to be due to impurities in the sample. The main weight loss associated with a smooth endotherm occurs over the range 270 to 350°C, indicating volatilisation of the intact compound, but with some decomposition indicated by the remaining residue.

The compound $Ba(HDFDD)_2$, Example 6, shows (Figure 9) a major weight loss due to volatilisation over the temperature range 280 to 350°C, but with considerable decomposition indicated by the weight of the residue remaining after volatilisation.

If desired the novel compounds of this invention can be complexed with a neutral oxygen or nitrogen donor ligand in accordance with the teaching of, and by the technique taught in EP-A-0405634, such complexes also falling within the scope of the present invention.

Suitable donor ligands for this purpose are organic compounds (Lewis bases) having at least one heteroatom, usually oxygen or nitrogen, but possibly also a phosphorus or sulphur atom, which heteroatom(s) carry at least one unshared pair of electrons capable of forming a donor ligand bound to the metal cation, thereby to form a donor ligand coordination complex in which the metal cation shows a 1-to 6-fold coordination with the donor ligand in the crystal lattice. As will be appreciated by those skilled in the art, the organic ligands will be relatively low molecular weight compounds of size to fit into the crystal lattice in 1- to 6-fold coordination with the metal cation.

Suitable donor ligands for this purpose are disclosed in EP-A-0405634 as well as methods for the formation of such complexes. Quite simply this method usually comprises suspending the uncomplexed compound in a suitable organic liquid suspension medium, e.g. toluene or xylene, adding the ligand to the suspension, stirring and optionally warming the dispersion to provide a clear solution, and finally cooling the solution, e.g. by refrigeration, to crystallise the coordination complex.

In the alternative, and preferably, the complexes are prepared by reacting a source of the metal, e.g. the elemental metal oxide, hydroxide, amide, alkoxide, hydride or metal alkyl, in the solid phase, and preferably solid oxide, hydroxide, amide or alkoxide, with the appropriate $\beta$-diketone under substantially anhydrous conditions in the presence of an organic solvent containing the ligand, preferably in the required stoichiometric amount.

Whilst any of the organic donor ligands described in EP-A-0405634 are suitable, the preferred organic donor ligands used herein include: glyme, diglyme, crown ethers such as the 18-crown-6 ether and dibenzo-18-crown-6 ether, and nitrogen containing donor ligands such as tetramethylethylene diamine (TMED), hexamethylphosphoramide (HMPA), pentamethylethylenetriamine (PMEDTA), dimethyl-2-oxo-pyrimidine (DMPU), and dimethylimidazolidinone (DMI).

At this point, it may be re-emphasised that, contrary to the teachings of EP-A-0405634, the presence of the ligand in the compounds of this invention is not always essential to volatility and stability, although the

presence of the ligand may in some cases give improved volatility. A prime example given hereinafter is in compounds based on TDFND, the preferred diketone, where a surprisingly high volatility is achieved without the ligand. The incorporation of such ligands in accordance with the teachings of EP-A-0405634 is therefore optional so far as the present invention is concerned, but at least in some cases is a preferred option.

The preparation of such complexes is illustrated by Examples 7 to 9.

EXAMPLE 7

Preparation of Tetraglyme Complex of $Ba(TDFND)_2$

The reaction was carried out as in preceding Example 4. The Schlenk tube was charged with barium hydride (2.754g, 19.8mmol), dry toluene ($20cm^3$) and dry tetraglyme ($CH_3O(CH_2CH_2O)_4CH_3$, $4.33cm^3$, 19.8mmol) before the cautious addition of TDFND (16.04g, 19.7mmol). Heating to 80 ° C and stirring during several hours were required before the purple slurry progressed to a cloudy yellow solution containing a small quantity of purple solids. Filtration yielded a cloudy yellow solution. Following removal of the toluene under vacuum at room temperature, several cycles of freezing in liquid nitrogen and warming to room temperature, still under vacuum, produced a mass of yellow crystals within a yellow oil. This material was dissolved in hexane ($20cm^3$) and refrigerated overnight (-20 ° C) to produce a mass (17.63g, 76%) of pale yellow crystals in a small quantity of light brown liquor. The use of tetraglyme of high purity (Aldrich, >99%) was found to be important to the recovery of crystalline material.

Analysis, found C 28.80%, H 1.97%;

Calculated for $Ba(TDFND)_2$.tetraglyme, C 28.65%, H 2.04%.

EXAMPLE 8

Preparation of Other Lewis Base Complexes of $Ba(TDFND)_2$

Using the methods described above (Example 7), tetraglyme was replaced in separate experiments in the synthesis with stoichiometric quantities of alternative Lewis base donors. Single equivalents of 18-crown-6 and dibenzo-18-crown-6 and two equivalents of diglyme ($CH_3O(CH_2CH_2O)_2CH_3$) were employed. The crown ethers produced crystals on refrigeration of the initial toluene solution. The diglyme complex was recovered by reducing the solution in volume under vacuum until crystallisation began to occur, warming to redissolve, then refrigeration. Good yields of crystalline materials were obtained. Melting points of these materials were sharp, and different to that of the non-complexed material.

EXAMPLE 9

Preparation of Lewis Base Complexes of Other Highly Fluorinated $\beta$-diketonates

Using the methods described above (Example 7) 18-crown-6 and tetraglyme complexes of EF3D and HDFDD were prepared. In both cases, the 18-crown-6 complex was readily recovered by refrigeration of the toluene solution. Both tetraglyme complexes were recovered as oils which remained liquid down to -100 ° C. Characterisation was limited to that obtained from the STA experiment, the data was consistent with the proposed formulations.

STA traces of the above complexes are given in Figures 10 to 16.

As seen in Figure 10, the material $Ba(TDFND)_2$.tetraglyme (Example 7) shows the features associated with a melting point beginning at about 70 ° C. There is then a gentle endotherm, beginning at about 180 ° C and associated with a loss in weight. The endotherm ends only as the weight of the sample falls to near zero. This therefore represents volatilisation of an intact species, not accompanied by decomposition.

The traces shown in Figure 11 for the material $Ba(TDFND)_2$.18-crown-6 (Example 8) show a melting point onsetting at about 165 ° C, and a volatilisation over the range of about 210 to 340 ° C. This material leaves a moderate residue.

The traces (Figure 12) for the material $Ba(TDFND)_2$.2(diglyme) (Example 8) show a sharp endotherm, onset about 80 ° C, not accompanied by any weight loss. This is assigned to the melting point. There is then a second, more gradual endotherm over the range 100 to 250 ° C, corresponding to a loss of 28wt%. There is then a final, gentle endotherm starting at about 260 ° C and ending abruptly at just over 300 ° C as the weight of the sample falls to near zero. This may be explained as follows. The minimal residue shows that all products are volatile. The second exotherm is probably due to loss of diglyme (bpt 162 ° C, 22wt% of

molecule) and the third to evaporation of the intact $Ba(TDFND)_2$ left. Evidently, the weight loss over the range 100 to 250 °C is in part due to volatilisation of intact $Ba(TDFND)_2$.2(diglyme) and to $Ba(TDFND)_2$ - (onset 190 °C).

The traces (Figure 13) for the $Ba(EF3D)_2$.18-crown-6 complex (Example 9) show a sharp melting point onsetting at about 95 °C. The weight loss appears to occur in a single step over the range 200 to 350 °C to a modest residue. The break in the DTA curve at just over 300 °C indicates, however, that some exothermic decomposition occurs at this point.

The traces (Figure 14) for the $Ba(HDFDD)_2$.18-crown-6 complex (Example 9) show an endothermic weight loss (220 to 270 °C) in advance of the volatilisation (280 to 320 °C), believed to be due to contamination of the sample with uncomplexed 18-crown-6.

The traces (Figure 15) of the $Ba(EF3D)_2$.tetraglyme complex (Example 9) show only a modest residue. There is, however, evidence for at least two decompositions, the first possibly involving loss of tetraglyme, in advance of the main volatilisation.

Finally, the traces (Figure 16) for the $Ba(HDFDD)_2$.tetraglyme complex (Example 9) show that the complex behaves in similar fashion to the EF3D analogue, but leaves a more substantial residue.

In a further aspect of the present invention, coordination complexes of the novel compounds of this invention, and coordination complexes of the type disclosed in EP-A-0405634, are advantageously prepared by the reaction of a strongly basic source of the metal and solid metal oxide, hydride hydroxide, amide or alkoxide with the stoichiometric amount of the β-diketone in solution in an aromatic hydrocarbon, preferably toluene, containing an approximately stoichiometric amount of the organic donor ligand, to form a suspension, stirring the suspension, optionally with gentle warming, until the metal oxide, hydride hydroxide, amide or alkoxide dissolves in the reaction medium, and then cooling the solution, preferably by refrigeration to crystallise out the product complex.

In accordance with this aspect of the present invention, metal β-diketonate donor ligand complexes of the formula

$$M\{R^3C(O)CHC(O)R^4\}_x.pL$$

where

M is a metal cation of valency x, preferably an alkaline earth metal cation or rare earth metal cation;

$R^3$ and $R^4$ each independently represent $C_1$-$C_8$ alkyl, preferably fluoroalkyl, most preferably perfluoroalkyl;

L is an organic donor ligand (Lewis base); and

p is an integer of from 1 to 6;

are prepared by reacting a strongly basic source of the metal M, e.g. a solid metal hydride oxide, hydroxide, amide, or alkoxide, with the stoichiometric amount of a β-diketone of the formula:

$$R^3C(O)CH_2C(O)R^4$$

the reaction being carried out under anhydrous conditions in the presence of an aromatic hydrocarbon ligand, preferably toluene, containing the organic donor ligand (Lewis base) in the approximately stoichiometric amount (±10%), and recovering the product coordination complex from the reaction mixture. In this way the complexes are obtained in what is essentially a one-step process.

Where $R^3$ and $R^4$ respectively have the values defined herein for $R^1$ and $R^2$, i.e. $R^3 = R^1 = C_1$-$C_8$ perfluoroalkyl, preferably $-CF_3$ or $-nC_3F_7$, and $R^4 = R^2 = C_2$-$C_8$ perfluoroalkyl, preferably $-nC_3F_7$ to $-nC_6F_{13}$, this represents a convenient route to the preparation of the donor ligand complexes of the present compounds, i.e. compounds of the formula:

$$M\{R^1C(O)CHC(O)R^2\}_x.pL,$$

e.g. the route:

$$Ba(OH)_2 + 2\text{-}n\text{-}C_3F_7C(O)CH_2C(O)n\text{-}C_3F_7 + pL \xrightarrow[\text{+ refrigerate}]{\text{toluene}}$$

solid TDFND

$$Ba\{n\text{-}C_3F_7C(O)CHC(O)n\text{-}C_3F_7\}_2.pL$$

In the alternative, this represents an alternative and convenient route into the donor ligand complexes disclosed in EP-A-0405634, or for that matter the crown ligand complexes disclosed in EP-A-0460627, e.g. in the case where $R^3 = R^1 = C_1\text{-}C_8$ alkyl, preferably fluoroalkyl, preferably $CF_3$ or $n\text{-}C_3F_7$; $R^4 = R^2 = C_1\text{-}C_8$ alkyl, preferably fluoroalkyl, preferably $CF_3$ or $n\text{-}C_3F_7$, the route

$$M(OH)_2 + 2\{R^3C(O)CH_2C(O)R^4\} + pL \xrightarrow[\text{2) refrigerate}]{\text{1) toluene}}$$

solid.M = Ca.Ba.Sr ß-diketone ligand

$$M\{R^3C(O)CHC(O)R^4\}_2.pL$$

crystalline metal ß-diketonate donor ligand complex.

This route to the donor ligand complexes of EP-A-0405634 and EP-A-0460627 as well as donor ligand complexes of the present compounds is illustrated by the following Examples 10a to 10c:

## EXAMPLE 10a

### Direct Preparation of Lewis Base Complexes of Alkaline Earth Metal Bis($\beta$-diketonates)

An oven dried Schlenk tube was cooled during entry to the dry-box. It was there charged with solid barium hydroxide, $Ba(OH)_2.H_2O$(1.89g, 10mmol) and fitted with stopper and stirrer-bar. On removal from the dry-box, toluene (35cm$^3$), 1,3-dimethylimidazolidinone (DMI) (6.85g, 6.54cm$^3$, 60mmol) and 1,1,6,6-tetramethylheptanedione (TMHD) (3.68g, 4.18cm$^3$, 20mmol) were added by syringe against nitrogen flush. This reaction mixture was stirred at 80°C for one hour during which time a clear solution formed. Large, colourless cubic crystals were obtained by filtration and subsequent refrigeration (-20°C) of this solution. The crystals were separated by filtration, washed with hexane, dried and isolated.

$^1H$ nmr gave no evidence for the presence of water in the complex and was consistent with formulation as a DMI complex of $Ba(TMHD)_2$. Simultaneous Thermal Analysis also indicated presence of a volatile, labile ligand lost prior to volatilisation of the $Ba(TMHD)_2$. Elemental analysis was consistent with proposed formulation as $Ba(TMHD)_2.2DMI$, mp 94 to 95°C.

## EXAMPLE 10b

In another experiment, using a similar procedure, solid $Ba(OH)_2.H_2O$ (1.89g, 10mmol) was reacted with 1,1,1,5,5,5,-hexafluoropentane-2,5-dione (HFAcAc) (4.16g, 2.83cm$^3$, 20mmol) in the presence of tetraglyme (2.22g, 2.2cm$^3$, 10mmol) in toluene (20cm$^3$). The suspension cleared and became perceptibly warm to the touch during addition of the diketone. A quantity of water was observed to separate. After about twenty minutes stirring, a clear rose-pink solution had formed, the coloration presumably resulting from an impurity in the HFAcAc, above a small, clear aqueous layer. The water was removed by syringe and the solution filtered. A pink-tinged microcrystalline powder was recovered by removal of toluene in vacuo.

The powder gave a near identical STA trace to a previously authenticated sample of $Ba(HFAcAc)_2$.tetraglyme prepared using $BaH_2$. The melting point was broader and onset slightly lower consistent with the presence of a slight impurity. Elemental analysis and nmr were consistent with this formulation.

EXAMPLE 10c

Following the procedure of Example 10b, solid barium hydroxide is reacted with n-$C_3F_7C(O)CH_2C(O)$n-$C_3F_7$ in place of the HFAcAc. The suspension is stirred with gentle warming until a clear solution is obtained. Refrigeration of the solution yields the crystalline coordination complex

Ba{n-$C_3F_7C(O)CHC(O)$-n-$C_3F_7$}$_2$.tetraglyme

identical with the product of Example 7.

As already indicated, the novel alkaline earth metal and rare earth metal β-diketonates of this invention, optionally but not necessarily in the form of an organic donor ligand complex in accordance with the teachings of EP-A-0405634, are useful volatile metal sources for CVD processes, also known as Metallo-Organic Chemical Vapour Deposition (MOCVD) processes. In such processes a solid substrate, e.g. metal, glass, ceramic metal oxide or oxide mixture, e.g. MgO (sapphire) or yttrium stabilized zirconia, strontium titanate, or a semiconductor material, e.g. Si, is contacted under low pressure, high temperature conditions with the volatile metallo-organic compound or complex in vapour phase. Under these conditions, films containing metal deposit as a series of layers on the surface of the substrate, the growth of which is dependent amongst other things on the decomposition rate of the volatile metallo-organic compound in the vapour phase. Depending also on the presence of other components, e.g. oxygen gas, water vapour, in the vapour phase, the metal may deposit on the substrate either as an oxide or as a fluoride. Not only that, but the MOCVD vapour will often comprise a mixture of volatile organo-metallic compounds including volatile compounds of one or more of copper, yttrium, bismuth, thallium and lead, as well as one or more volatile alkaline earth metal and/or rare earth metal sources, so as to build up on the surface of the substrate by CVD mixed oxide and/or fluoride layers comprising a variety of different metal oxides and/or fluorides, especially those mixtures providing a deposited oxide layer with the properties of a superconductor.

The technical literature relating to MOCVD applications and techniques is extensive and replete with examples of MOCVD processes using volatile alkaline earth metal and rare earth β-diketonates and other volatile organo-metallic compounds of alkaline earth and rare earth metals and copper. Except insofar as the present invention concerns the use of the present β-diketonates in such MOCVD processes, the MOCVD processes as such form no part of the present invention, nor do they need be described here in more detail, since the use of the present compounds in those processes will be in accordance with established MOCVD procedures.

Further properties of the present compounds and complexes are illustrated in the following Examples.

EXAMPLE 11

Vacuum Sublimation of Ba(TDFND)$_2$.tetraglyme

A Schlenk tube was charged with Ba(TDFND)$_2$.tetraglyme (500mg). It was then fitted with a water-cooled cold finger, and evacuated to about 0.5 mBar. The apparatus was then placed in an oil bath held at about 150 °C, with vacuum maintained at 0.5 mBar. The off-white solids rapidly melted to a yellow oil. Within about thirty minutes, the cold finger was crusted with a white sublimate and a minimal brown residue remained at the bottom of the tube. Some 480 mg of white product was recovered. The STA trace of this material was essentially identical to that of non-sublimed material.

EXAMPLE 12

Vacuum Sublimations of Other Ba Bis(β-diketonate).Lewis Base Complexes

A series of vacuum sublimation trials were carried out as detailed above. The results are summarised in the table below.

| Complex | Time (min) | Sublimate (mg) | Residue (mg) | STA traces of Sublimate and Residue |
|---|---|---|---|---|
| TDFND | | | | |
| 2(diglyme) | 420 | 400 | 100 | Both distinct and neither identical to parent complex |
| 18-crown-6 | 180 | 450 | 50 | Identical to parent |
| Dibenzo-18-crown-6 | 120 | 60 | 400 | Sublimate lower melting |
| EF3D | | | | |
| Non-complexed | 60 | 100 | 400 | Both are oils. Sample decomposes |
| 18-crown-6 | 75 | 290 | 210 | Sublimate has fractionally lower mpt |

EXAMPLE 13

Carry-Over Rates for $Ba(TDFND)_2$

One of four lines in a CVD deposition system featuring standard gas handling facilities was used for these experiments. In this system, the precursor pot was heated by circulating hot air in a separate, temperature-controlled enclosure. The gas line was fitted with valves allowing the flow to be vented to exhaust or to have direct entry to the reactor. This allowed conditions to be stabilised before deposition was attempted. All components of the system between precursor container and exhaust were heated to a temperature 20°C above the precursor pot temperature to prevent premature condensation. Carry-over rates were determined between deposition runs, i.e. with previously stabilised precursor flows. The gases were passed through a cold (ambient temperature) trap between the system and the rotary pump. The mass of solids trapped per unit time was determined during seven runs over the space of seven days. A system pressure of 10 Torr was used, at an argon flow rate of 200 sccm. The precursor pot temperature was 161 ± 1°C.

| Run day | Carry-Over Rates (micrograms per minute) |
|---|---|
| 1 | 190, 180, 180 |
| 4 | 160, 190 |
| 7 | 177, 175 |

This gives a carry-over rate of 179 ± 20 $ugmin^{-1}$. Within the limits of the experiment this represents a reproducible carry-over rate suitable for carrying out MOCVD.

The utility of the present compounds and complexes in MOCVD applications is illustrated by the following Examples.

EXAMPLE 14

Deposition Rate of $BaF_2$ from $Ba(TDFND)_2$

The precursor delivery system described above was utilised. The gases were admitted into an impinging jet reactor (Archer Technicoat Limited) with a resistively heated substrate platform (U.S. Inc.). Deposition was carried out onto silicon substrates of (100) surface orientation at a wafer surface temperature of 661 °C (platform temperature of 750 °C). Other conditions were as for the carry-over runs. Film thickness was measured using an ellipsometer. A total of nineteen runs were accumulated, over a period of 67 days.

| Day No. | Deposition Rate, j in $Åmin^{-1}$ |
|---|---|
| 1 | 29.1, 28.8, 29.3, 29.3 |
| 32 | 27.8 |
| 33 | 30.3, 32.9 |
| 54 | 31.2, 26.8, 31.3, 29.6 |
| 64 | 26.8, 28.8, 28.7, 27.2 |
| 67 | 30.9, 31.6, 32.3, 28.3 |

This represents an average deposition rate of, j, of 29.49 ± 3.62 $Åmin^{-1}$. Long term stability in use and an acceptable reproducibility for MOCVD purposes of deposition rate are thus demonstrated.

EXAMPLE 15

Characterisation of $BaF_2$ Deposited from $Ba(TDFND)_2$

The conditions and apparatus used above were further used to deposit $BaF_2$ on silicon (100). The crystalline phases present and their orientation within the film were characterised using an X-ray diffractometer with $CuK\alpha$ radiation. The strongly adherent film was found to be $BaF_2$ completely oriented with the (111) plane orthogonal to the substrate surface. In the diffractogram the lines for the (111) series out to (333) were readily identified.

EXAMPLE 16

Arrhenius Plot for $BaF_2$ Deposition

The techniques used above were employed to produce kinetic data for the deposition. The conditions described before were used to bring a flow of gas into the impinging jet reactor. The temperature of the substrate platform was varied. The Arrhenius plots (Growth Rate in $Amin^{-1}$ as ordinate and 1000/T in $K^{-1}$ as abscissa) are given in Figures 17 and 18 for the heater platform temperature ranges 623 to 1023K and 623 to 673K, respectively. The transition between kinetic and mass-transfer limited growth can be seen in Figure 17. The linearity of both plots shows the reliability and in-use stability of the precursor.

## Claims

**1.** Metal $\beta$-diketonates of the formula

$M\{R^1C(O)CHC(O)R^2\}_x$

where
M is a metal cation of valency x,
$R^1$ is $C_1$-$C_8$ perfluoroalkyl
$R^2$ is $C_2$-$C_8$ perfluoroalkyl.

**2.** Metal $\beta$-diketonates according to claim 1, where M is a cation of an alkaline earth metal or rare earth metal.

**3.** Metal $\beta$-diketonates according to claim 2, where M is Ca, Ba, Sr or Y.

4. Metal β-diketonates according to any one of claims 1 to 3, where $R^1$ is $-CF_3$ or $n-C_3F_7$ and $R^2$ is $C_2-C_6$ perfluoro-n-alkyl.

5. Metal β-diketonates according to any one of claims 1 to 3, where $R^1$ and $R^2$ are both $n-C_3F_7$.

6. The compounds:
$Ca\{CF_3C(O)CHC(O)n-C_3F_7\}_2$
$Ba\{CF_3C(O)CHC(O)n-C_3F_7\}_2$
$Sr\{CF_3C(O)CHC(O)n-C_3F_7\}_2$
$Ca\{n-C_3F_7C(O)CHC(O)n-C_3F_7\}_2$
$Ba\{n-C_3F_7C(O)CHC(O)n-C_3F_7\}_2$
$Sr\{n-C_3F_7C(O)CHC(O)n-C_3F_7\}_2$
$Ca\{CF_3C(O)CHC(O)n-C_6F_{13}\}_2$
$Ba\{CF_3C(O)CHC(O)n-C_6F_{13}\}_2$
$Sr\{CF_3C(O)CHC(O)n-C_6F_{13}\}_2$
$Ca\{n-C_3F_7C(O)CHC(O)n-C_6F_{13}\}_2$
$Ba\{n-C_3F_7C(O)CHC(O)n-C_6F_{13}\}_2$
$Sr\{n-C_3F_7C(O)CHC(O)n-C_6F_{13}\}_2$
$Y\{CF_3C(O)CHC(O)n-C_3F_7\}_3$
$Y\{n-C_3F_7C(O)CHC(O)n-C_3F_7\}_3$.

7. Coordination complexes of the metal β-diketonates claimed in claim 1, being coordination complexes of the formula

$M\{R^1C(O)CHC(O)R^2\}_x.pL$

where
M, $R^1$, $R^2$ and x are as defined in claim 1;
L is an organic donor ligand (Lewis base); and
p is an integer of from 1 to 6.

8. Coordination complexes according to claim 7, where M, $R^1$ and $R^2$ are as defined in any one of claims 1 to 5.

9. Coordination complexes according to claim 7 or 8, where the organic donor ligand L (Lewis base) is an acyclic ether or polyether, a cyclic ether or polyether, or an organic N-containing donor ligand.

10. Coordination complexes according to claim 9, where the organic donor ligand is glyme, diglyme, tetraglyme, an 18-crown-6 cyclic ether, TMED, HMPA, PMDETA, DMPU or DMI.

11. A method of forming a metal-containing film on a substrate by chemical vapour deposition and which comprises contacting the substrate with a volatile fluorinated organo-metallic compound in the vapour phase and under conditions which cause the metal to deposit on the substrate either as a fluoride or oxide containing film, or a mixture of the two, including mixed oxide and mixed fluoride films of two or more different metals, depending upon the composition of the vapour phase, wherein there is used as a volatile organo-metallic component of said vapour a metal β-diketonate as claimed in any one of claims 1 to 6, or a coordination complex as claimed in any one of claims 7 to 10, or a mixture of two or more such β-diketonates and/or complexes.

12. A method according to claim 11, wherein the substrate is glass, metal, a ceramic oxide or oxide mixture, strontium titanate or a semiconductor material.

13. A method for the preparation of metal β-diketonate coordination complexes of the formula

$M\{R^3C(O)CHC(O)R^4\}_x.pL$

where
M and x are as defined in claim 1;

L and p are as defined in claim 7;

$R^3$ and $R^4$ are each independently selected from $C_1$-$C_8$ alkyl and $C_1$-$C_8$ fluoroalkyl;

which comprises reacting a solid hydride, oxide, hydroxide, amide or alkoxide of the metal M with the stoichiometric amount of a $\beta$-diketone of the formula

$R^3C(O)CH_2C(O)R^4$

under anhydrous conditions in the presence of an aromatic hydrocarbon solvent containing an approximately stoichiometric amount of the ligand L, and recovering the product coordination complex from the reaction mixture.

**14.** A method according to claim 13, wherein the product coordination complex is recovered in crystalline form by refrigeration of the reaction mixture and separation of the resulting crystalline product from the reaction solvent.

**15.** A method according to claim 13 or 14, wherein the metal reactant is an oxide or hydroxide.

**16.** A method according to any one of claims 13 to 15, wherein M is as defined in claim 2 or 3.

**17.** A method according to any one of claims 13 to 16, where L is as defined in claim 9 or 10.

**18.** A method according to any one of claims 13 to 17, wherein $R^3$ and $R^4$ respectively have the values as defined in claim 1 for $R^1$ and $R^2$, thereby to produce coordination complexes of the $\beta$-diketonates claimed in claim 1, viz coordination complexes of the formula

$M\{R^1C(O)CHC(O)R^2\}_x.pL$

where M, $R^1$, $R^2$ and x are as defined in claim 1, and L and p are as defined in claim 7.

**19.** A method according to claim 18, where $R^3$ and $R^4$ are groups as defined for $R^1$ and $R^2$, respectively, in either claim 4 or claim 5.

**20.** A method of forming a metal-containing film on a substrate by chemical vapour deposition and which comprises contacting the substrate with a volatile organo-metallic compound in the vapour phase and under conditions which cause the metal to deposit on the substrate either as a fluoride or oxide containing film, or a mixture of the two, including mixed oxide and mixed fluoride films of two or more different metals, depending upon the composition of the vapour phase, wherein there is used as a volatile organo-metallic component of said vapour a metal $\beta$-diketonate coordination complex obtained by the method claimed in any one of claims 13 to 19, or a mixture of two or more such complexes.

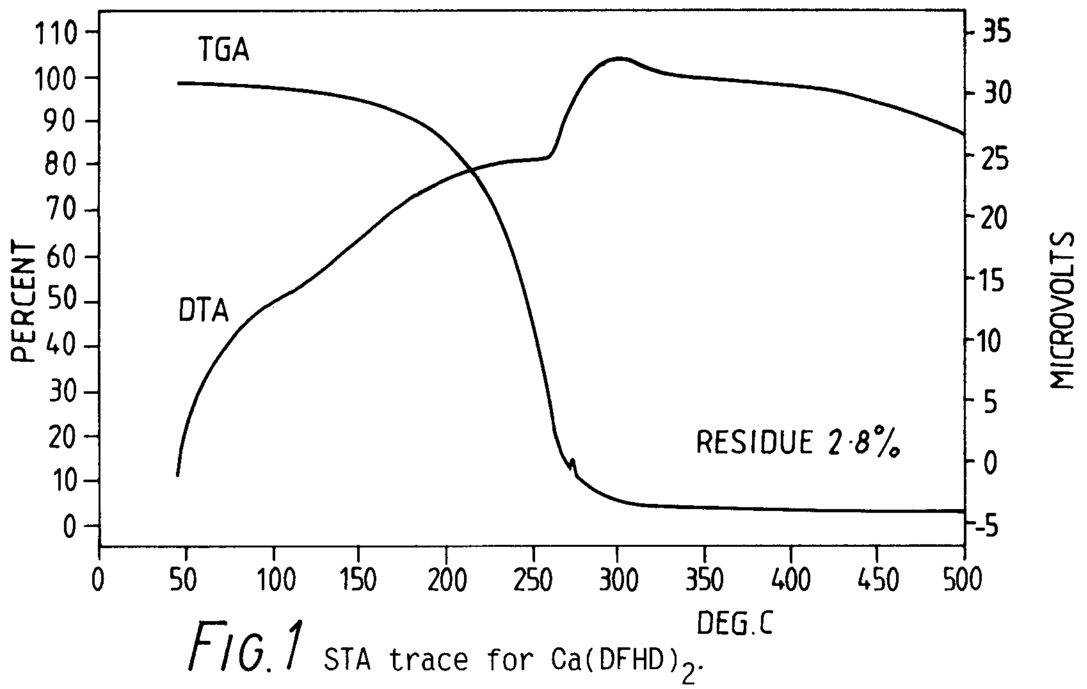


FIG. 1 STA trace for $Ca(DFHD)_2$.

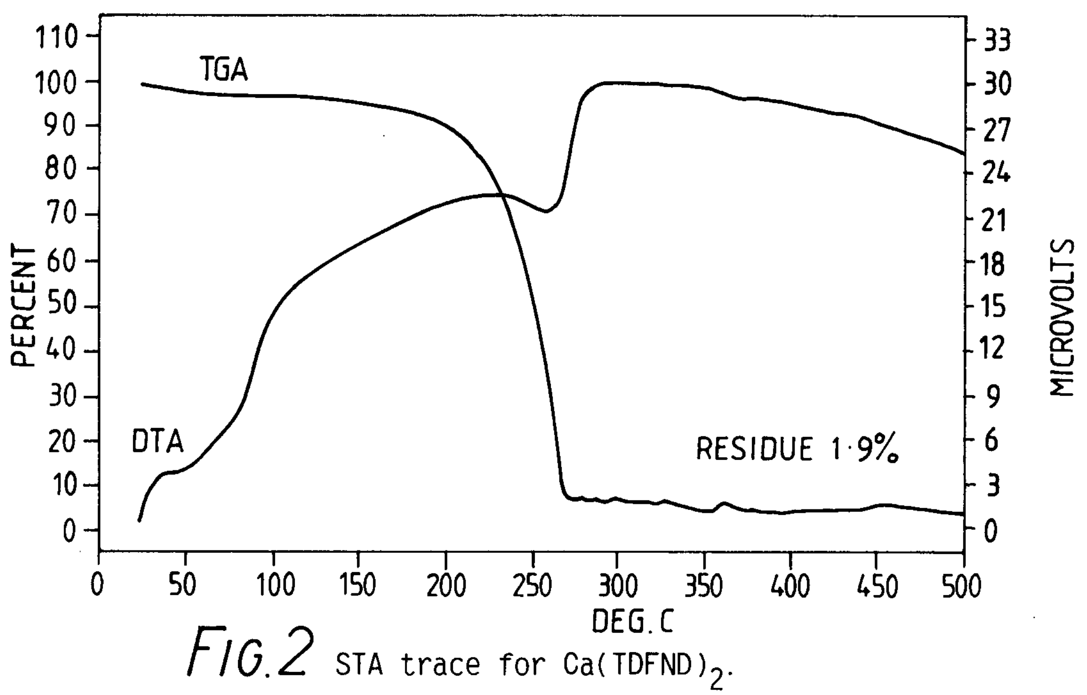


FIG. 2 STA trace for $Ca(TDFND)_2$.

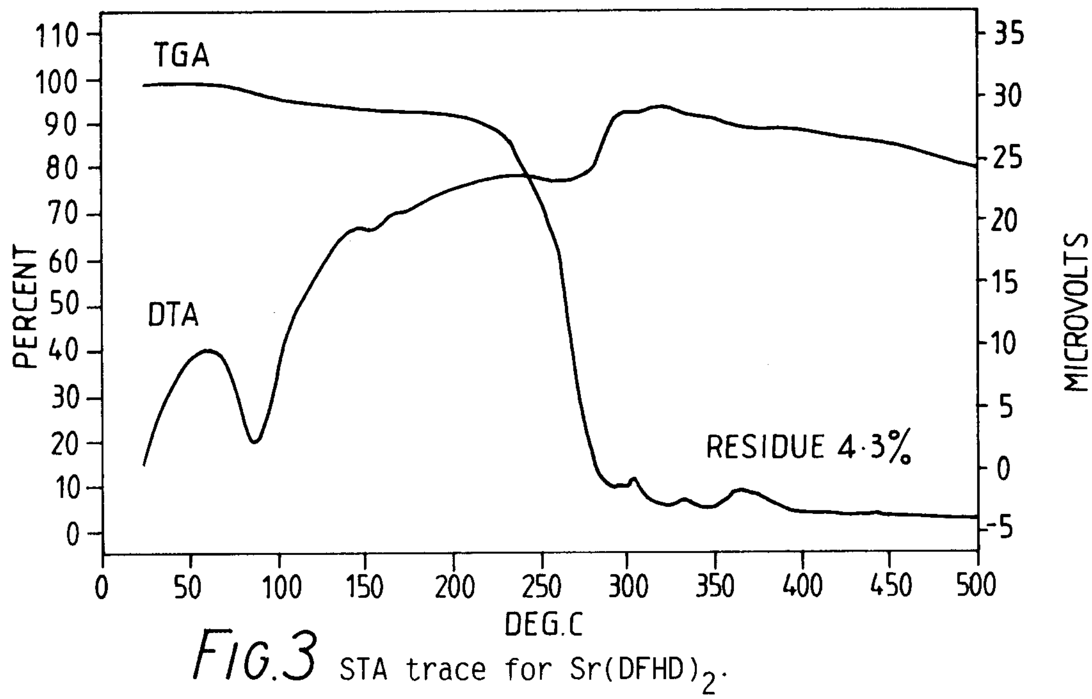


FIG.3 STA trace for $Sr(DFHD)_2$.

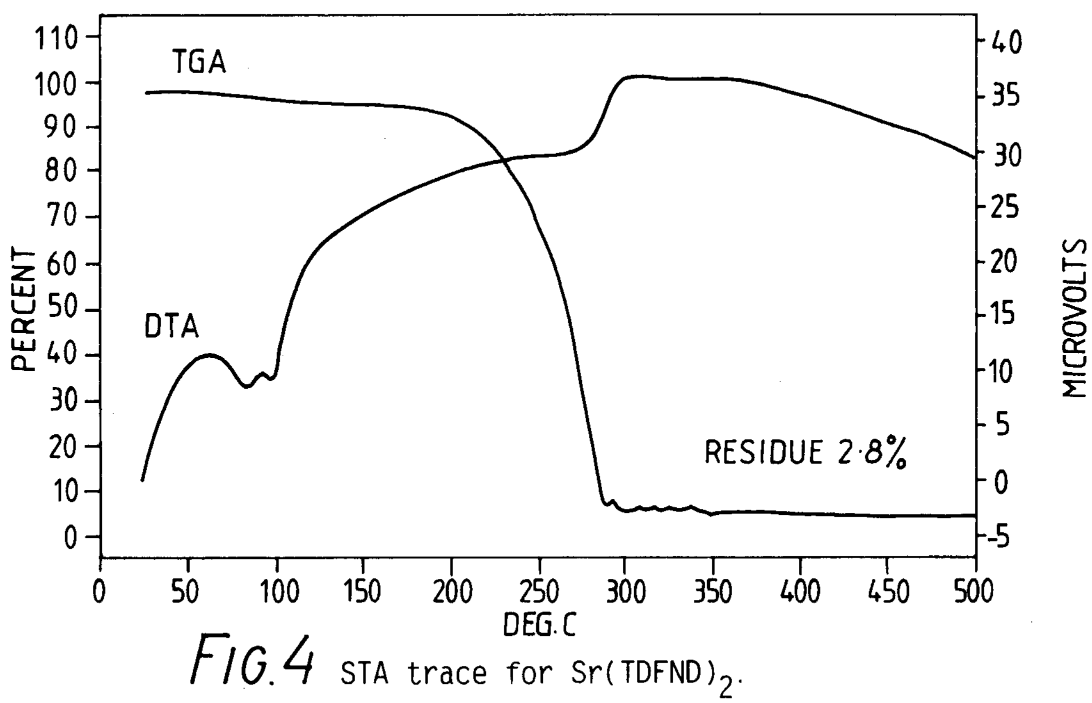


FIG.4 STA trace for $Sr(TDFND)_2$.

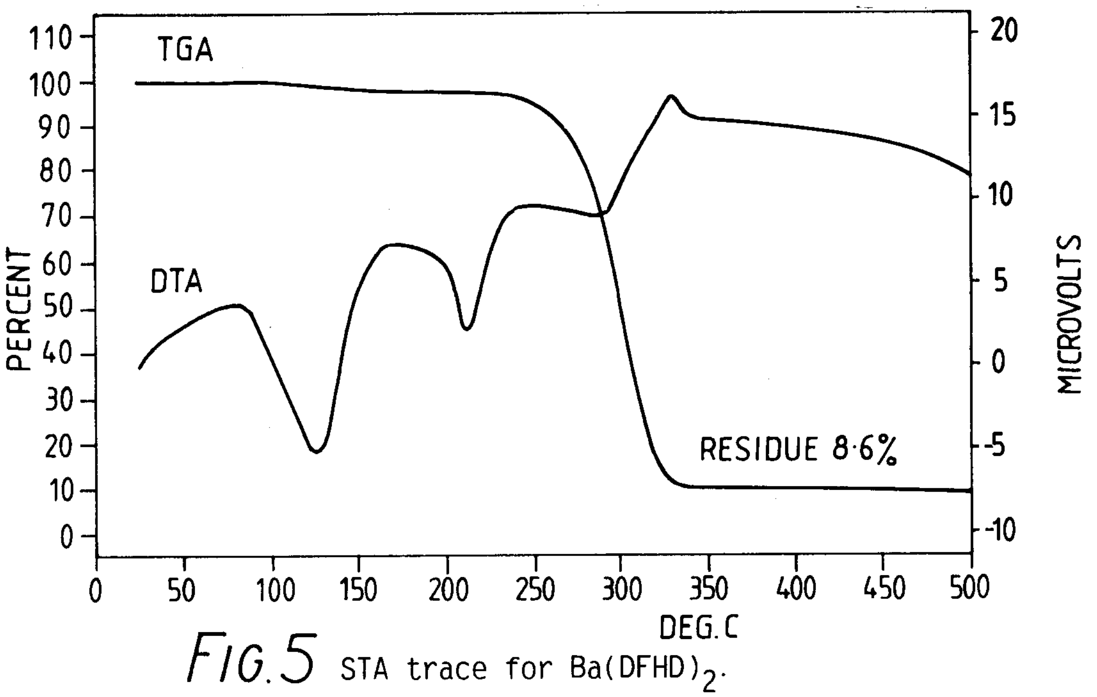


FIG.5 STA trace for Ba(DFHD)$_2$.

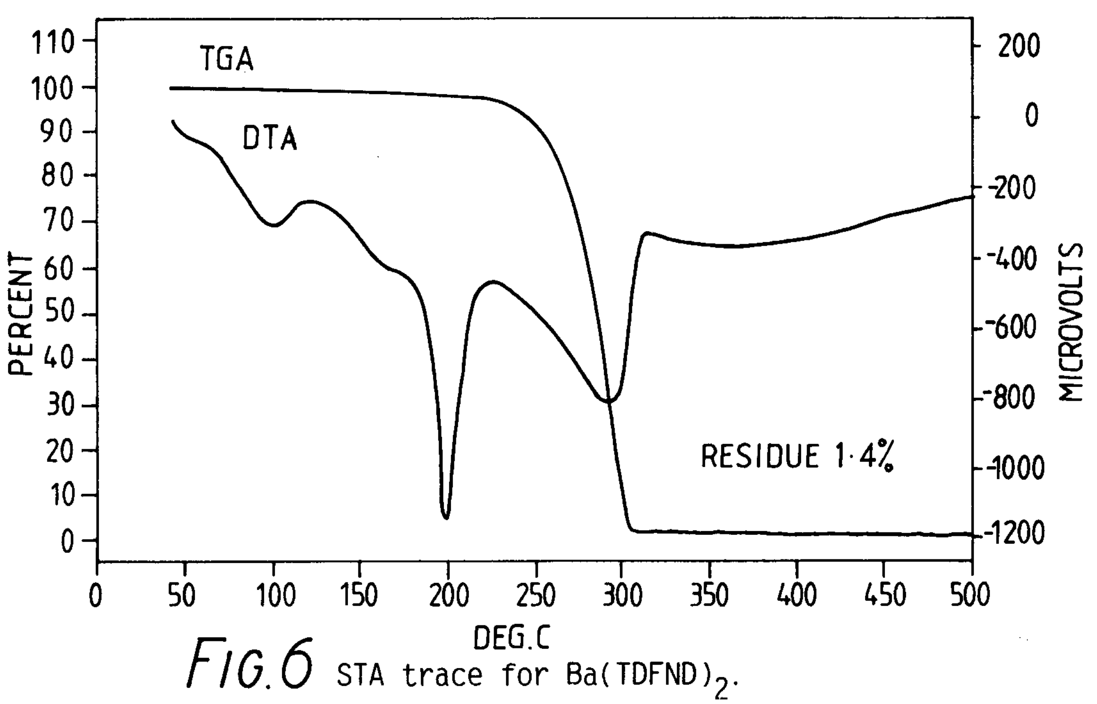


FIG.6 STA trace for Ba(TDFND)$_2$.

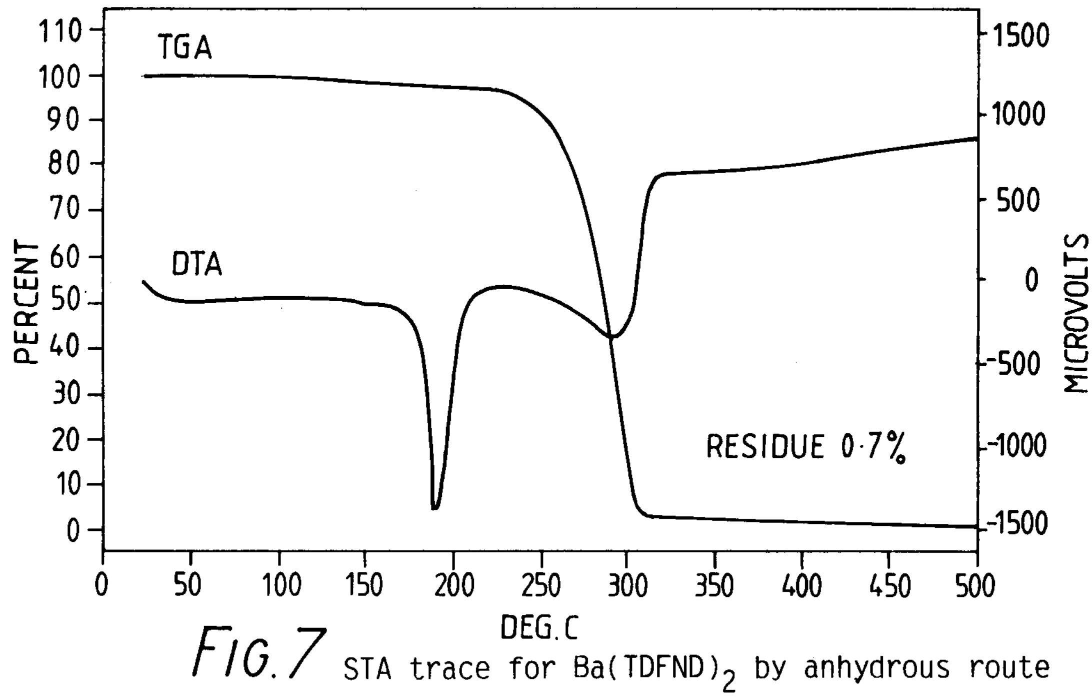


*FIG.7* STA trace for Ba(TDFND)$_2$ by anhydrous route

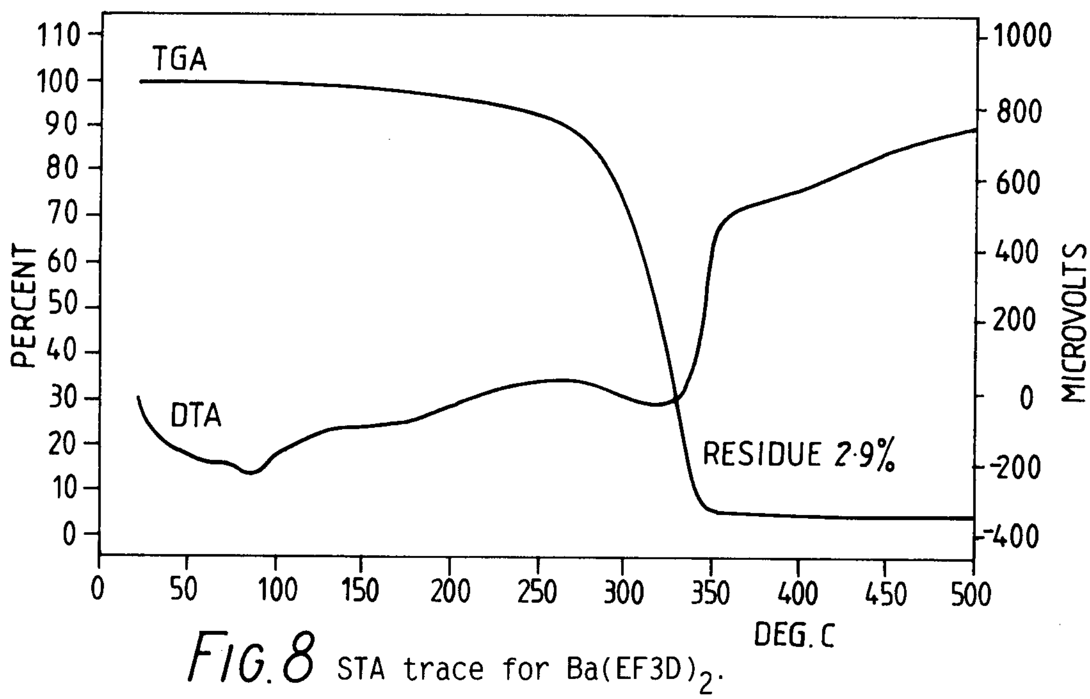


*FIG.8* STA trace for Ba(EF3D)$_2$.

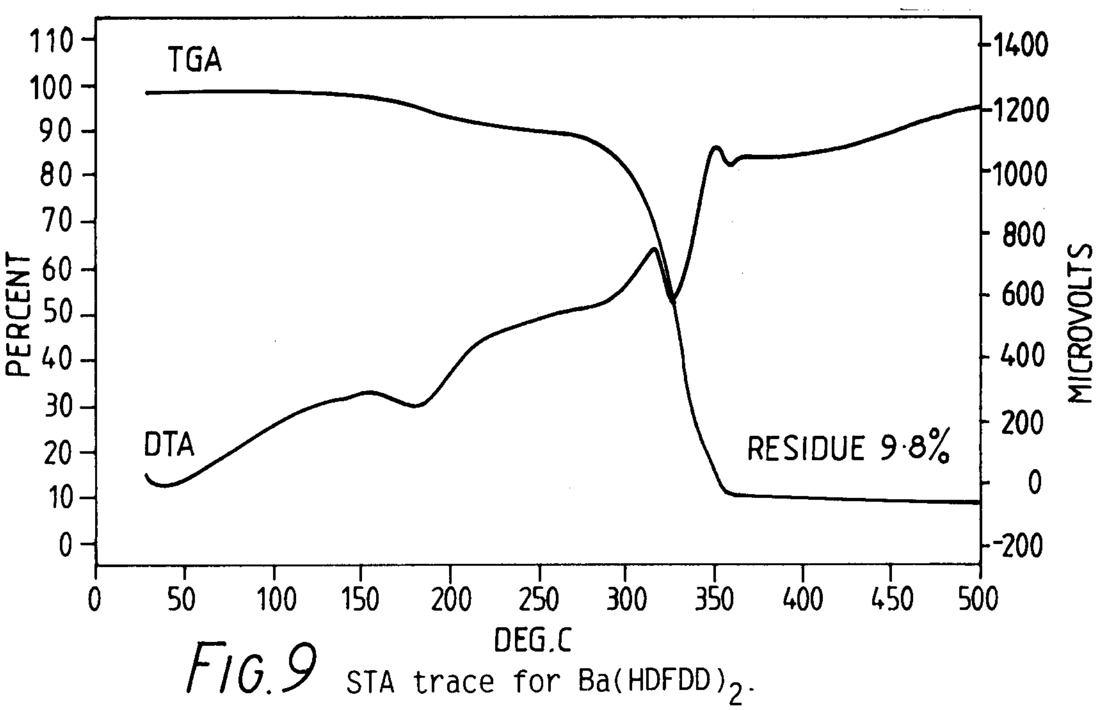


FIG.9 STA trace for $Ba(HDFDD)_2$.

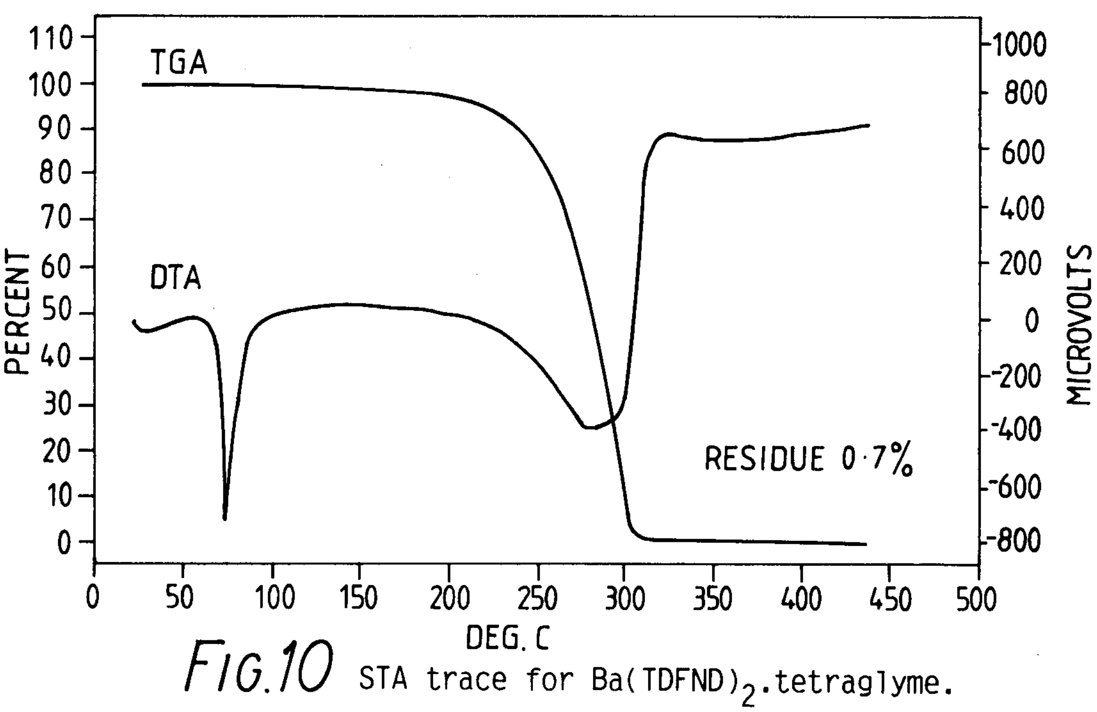


FIG.10 STA trace for $Ba(TDFND)_2$.tetraglyme.

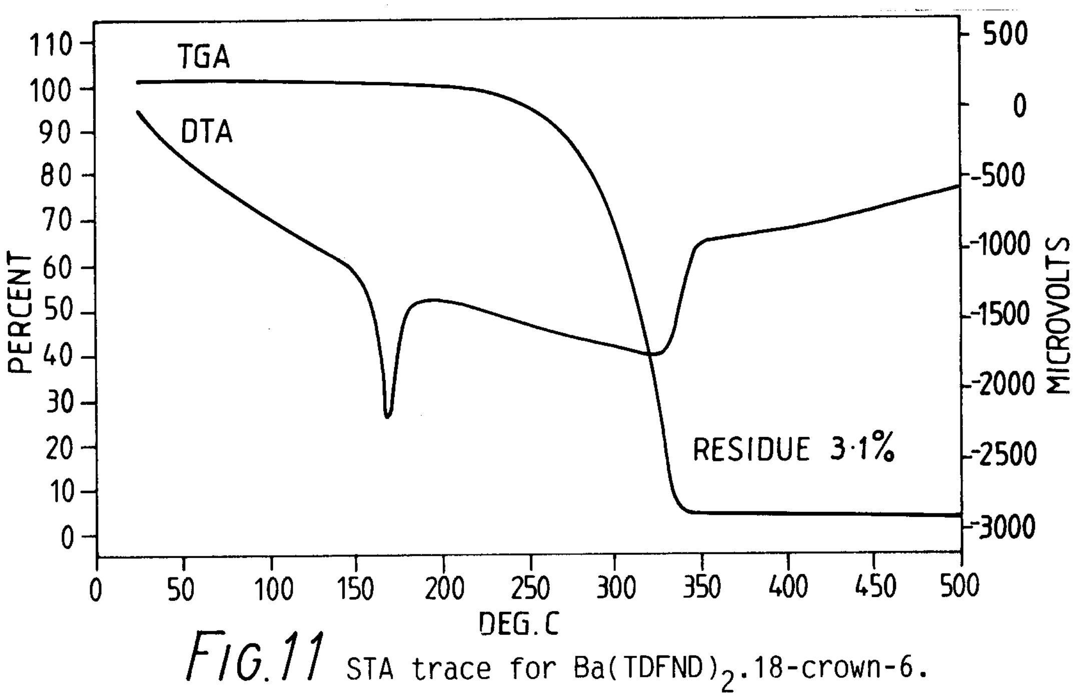


FIG.11 STA trace for $Ba(TDFND)_2$.18-crown-6.

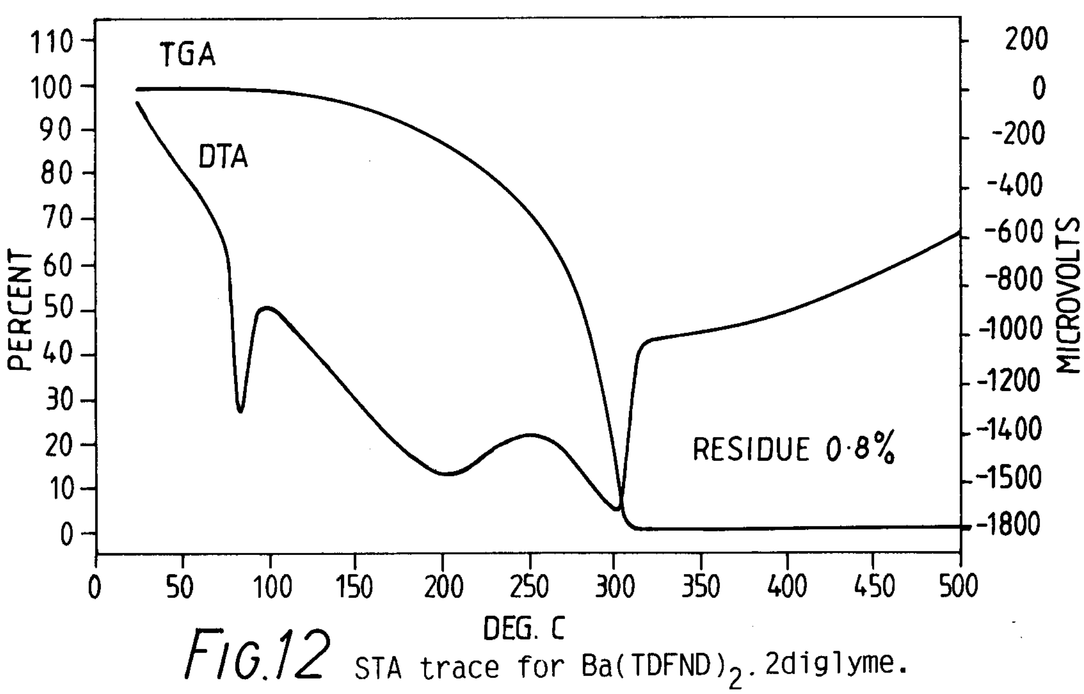


FIG.12 STA trace for $Ba(TDFND)_2$.2diglyme.

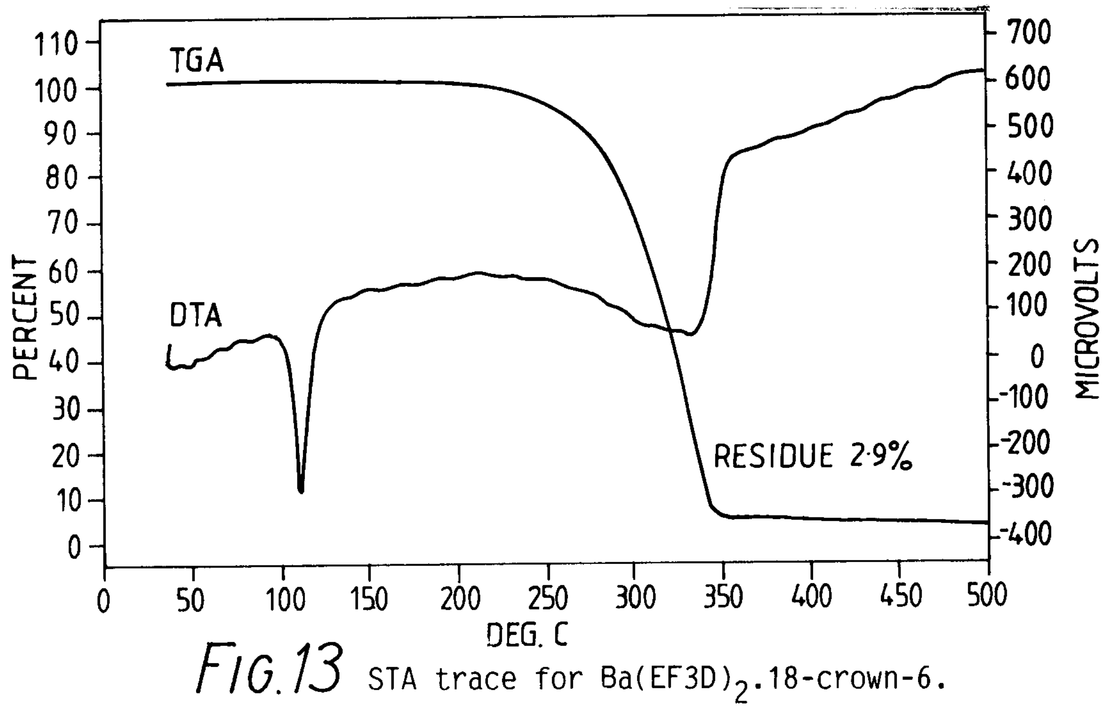


FIG. 13 STA trace for $Ba(EF3D)_2$.18-crown-6.

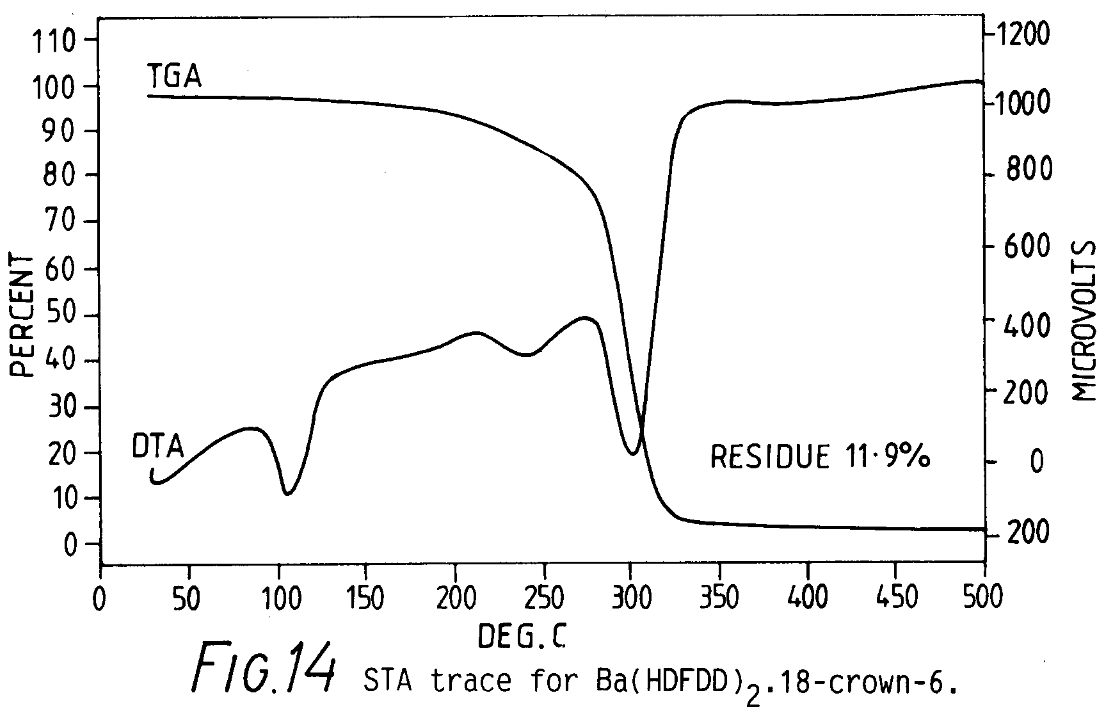


FIG. 14 STA trace for $Ba(HDFDD)_2$.18-crown-6.

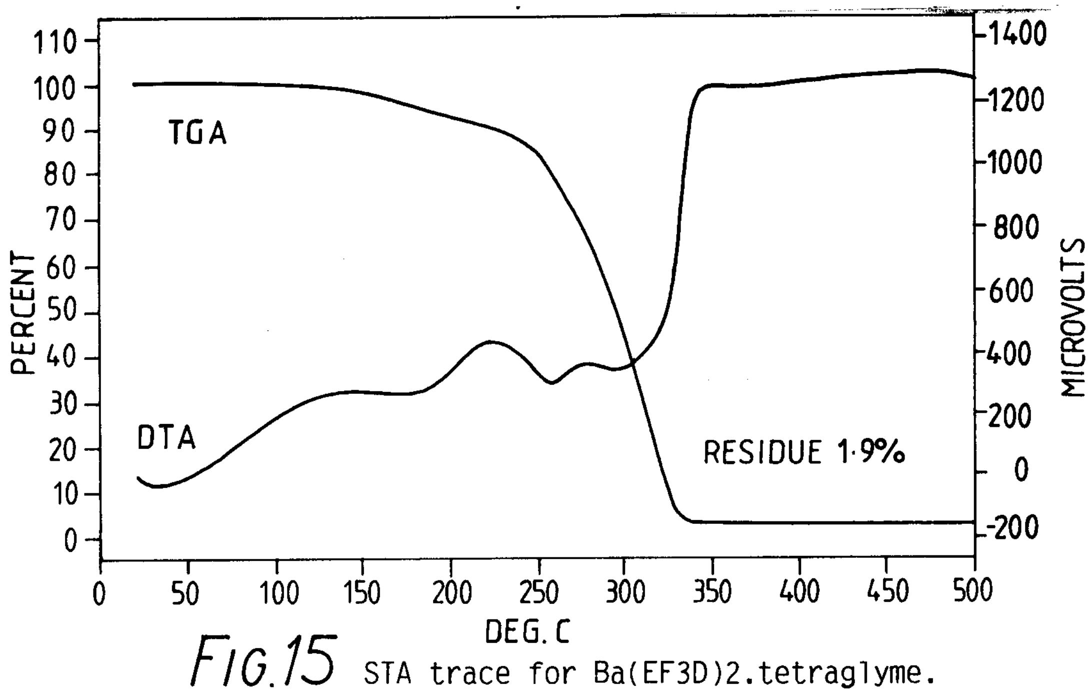


FIG. 15 STA trace for Ba(EF3D)2.tetraglyme.

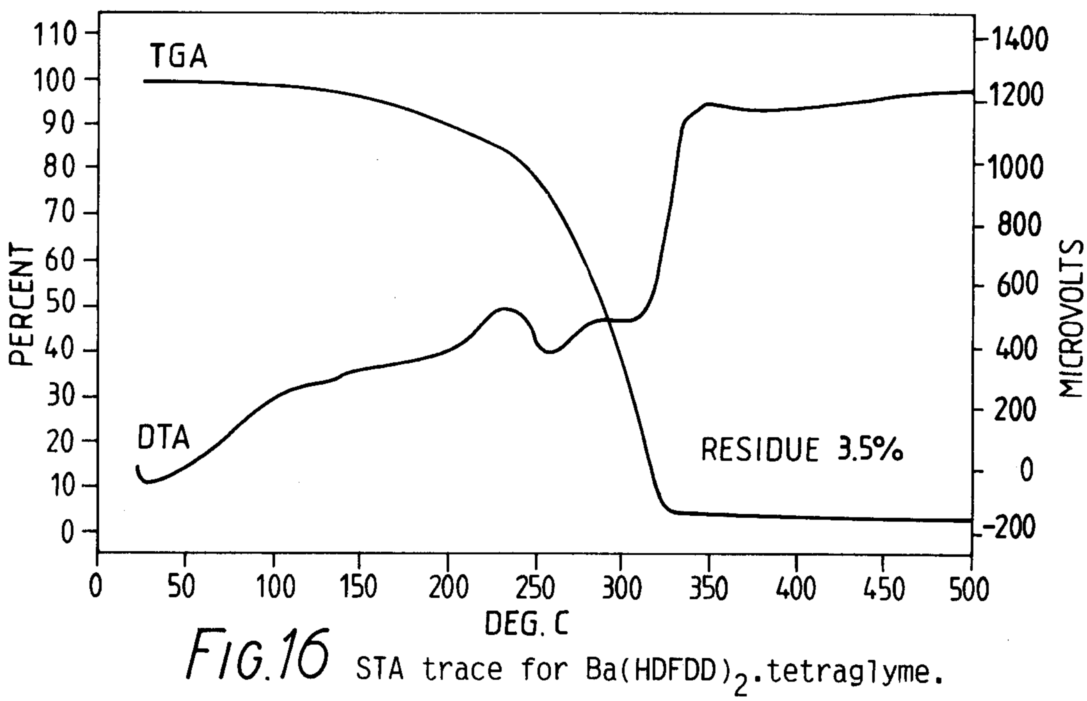


FIG. 16 STA trace for $Ba(HDFDD)_2$.tetraglyme.

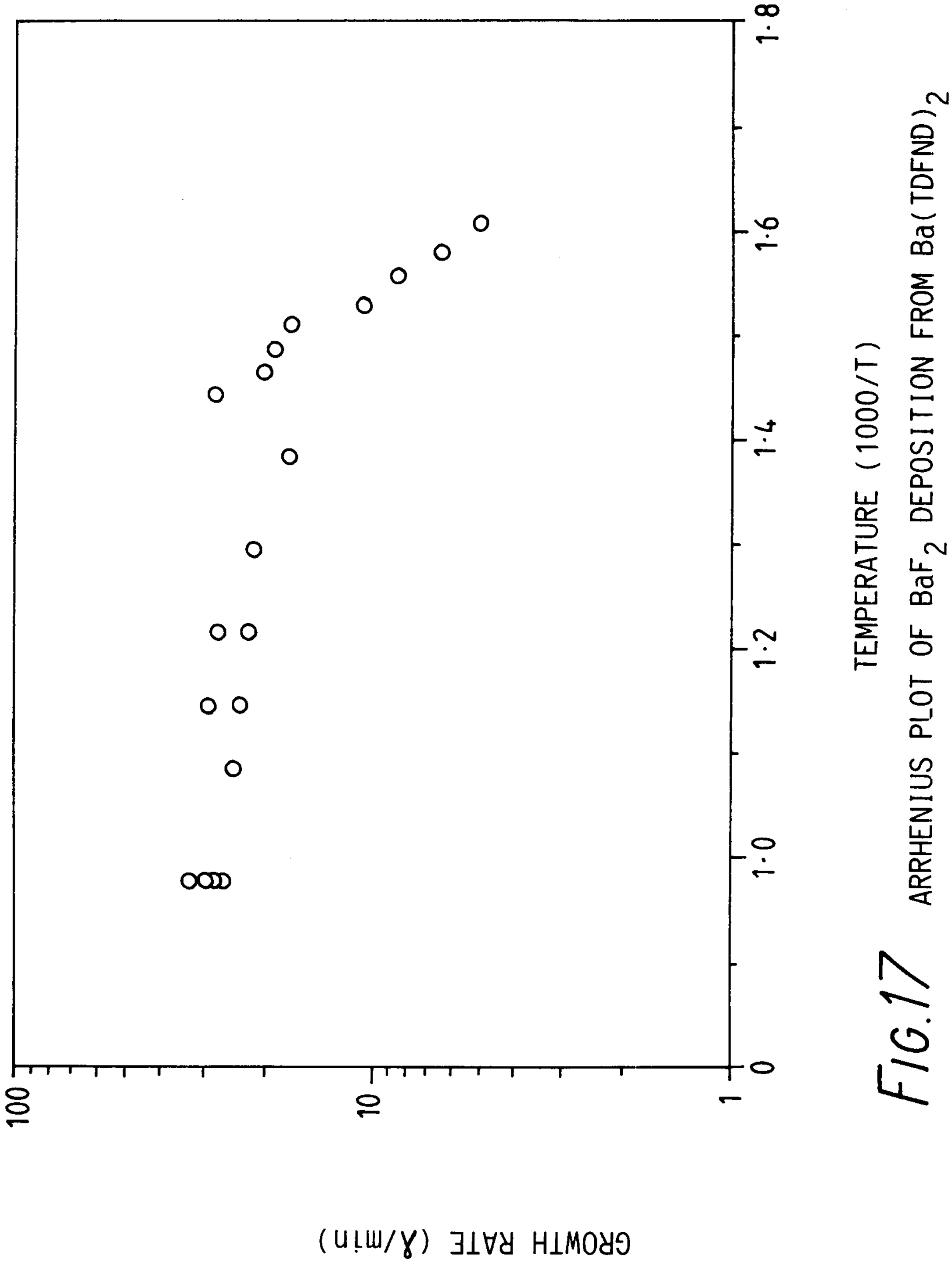


FIG.17 ARRHENIUS PLOT OF $BaF_2$ DEPOSITION FROM $Ba(TDFND)_2$

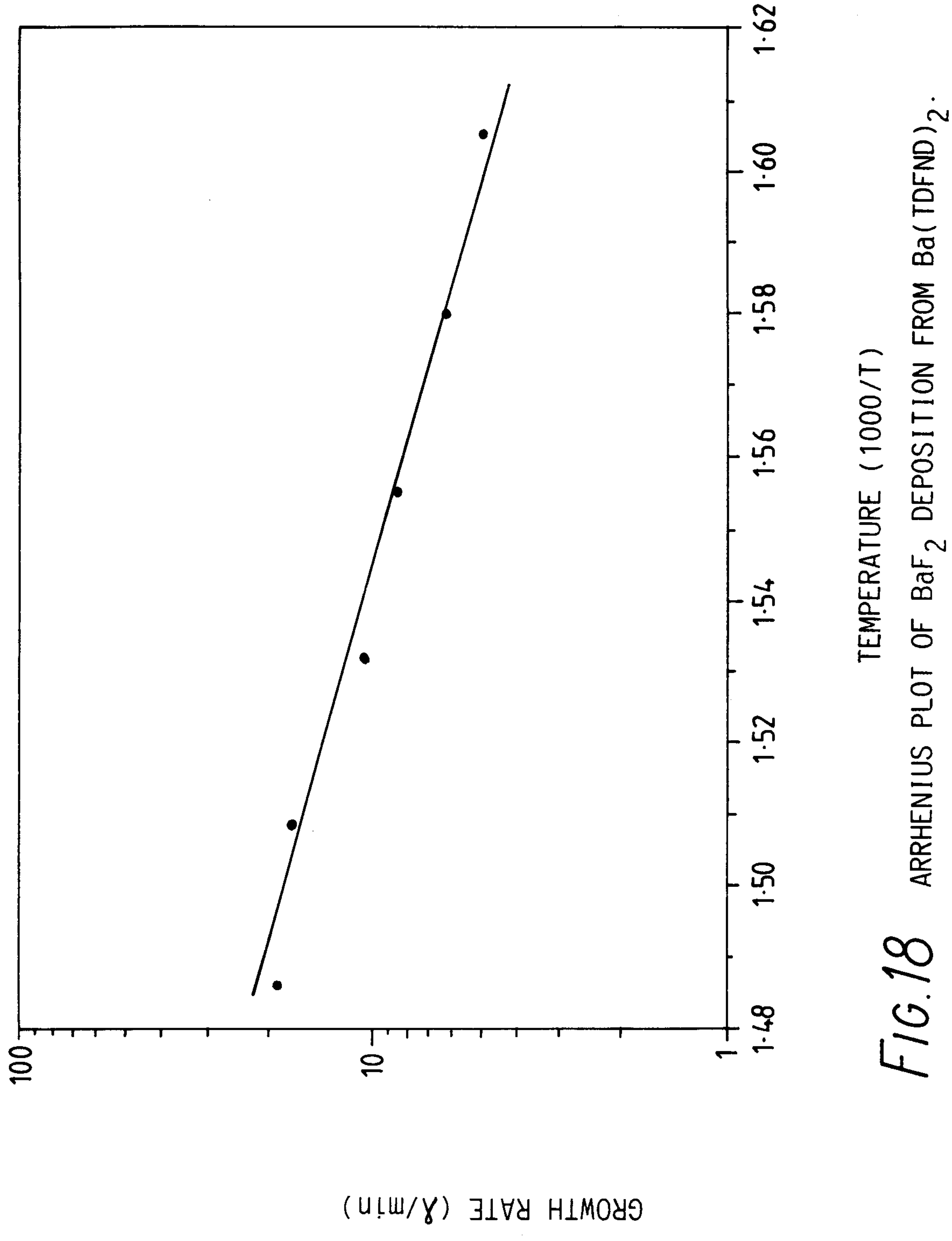


FIG. 18 ARRHENIUS PLOT OF $BaF_2$ DEPOSITION FROM $Ba(TDFND)_2$.

European Patent Office

# EUROPEAN SEARCH REPORT

Application Number

EP 92 30 7490
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,A | EP-A-0 405 634 (NEDERLANDSE ORGANISATIE VOOR TNO)<br>* the whole document *<br>--- | 1-20 | C07F3/00<br>C07F5/00<br>C07C49/92<br>C07C45/77<br>C23C16/00<br>//C07C49/167 |
| A | US-A-5 028 724 (IVANKOVITS, J. C. ET AL.)<br>* the whole document *<br>--- | 1-20 | |
| D,A | US-A-4 558 144 (FAY, R.C. ET AL)<br>* the whole document *<br>--- | 1-20 | |
| A | WO-A-8 907 666 (NORTHWESTERN UNIVERSITY)<br>* the whole document *<br>--- | 1-20 | |
| D,P,<br>X | EP-A-0 460 627 (AIR PRODUCTS AND CHEMICALS, INC.)<br>* the whole document *<br>--- | 1-20 | |
| X | INORGANIC CHEMISTRY<br>vol. 10, no. 3, 1971,<br>pages 498 - 504<br>RICHARDSON, M.F. ET AL<br>* the whole document *<br>--- | 1-10,<br>13-19 | TECHNICAL FIELDS SEARCHED (Int. Cl.5 ) |
| X | CHEMICAL ABSTRACTS, vol. 85,<br>1976, Columbus, Ohio, US;<br>abstract no. 158842r,<br>PICKERING, R.A. ET AL<br>page 450 ;<br>* abstract *<br>& J. MAGN. RESON.<br>vol. 22, no. 2, 1976,<br>pages 385 - 387<br>---<br>-/-- | 1,4,5 | C07F<br>C07C<br>C23C |

The present search report has been drawn up for all claims

| Place of search | Date of completion of the search | Examiner |
|---|---|---|
| THE HAGUE | 13 OCTOBER 1992 | RINKEL L.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 92 30 7490
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | CHEMICAL ABSTRACTS, vol. 69, 1968, Columbus, Ohio, US; abstract no. 110075k, SCHWEITZER, G.K. ET AL page 10323 ; * abstract * & ANAL. CHIM. ACTA vol. 43, no. 2, 1968, pages 332 - 334 --- | 1,4 | |
| X | CHEMICAL ABSTRACTS, vol. 92, 1980, Columbus, Ohio, US; abstract no. 155077d, SHAPOVALOV, V.A. ET AL page 547 ; * abstract * & ZH. OBSHCH. KHIM. vol. 50, no. 1, 1980, pages 103 - 107 --- | 1,4 | |
| X | CHEMICAL ABSTRACTS, vol. 73, 1970, Columbus, Ohio, US; abstract no. 92153k, SCRIBNER, W.G. page 309 ; * abstract * & KERTES, A.S. ED. 'SOLVENT EXTR. RES., PROC. INT. CONF. SOLVENT EXTR. CHEM., 5TH, 1968' 1969 , WILEY-INTERSCI. , NEW YORK --- | 1,4 | TECHNICAL FIELDS SEARCHED (Int. Cl.5 ) |
| | -/-- | | |

The present search report has been drawn up for all claims

| Place of search | Date of completion of the search | Examiner |
|---|---|---|
| THE HAGUE | 13 OCTOBER 1992 | RINKEL L.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 92 30 7490
Page 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | CHEMICAL ABSTRACTS, vol. 78, 1973, Columbus, Ohio, US; abstract no. 140690k, BURGETT, C.A. ET AL page 302 ; * abstract * & J. CHROMATOGR. vol. 77, no. 2, 1973, pages 265 - 276 | 1,4 | |
| X | CHEMICAL ABSTRACTS, vol. 85, 1976, Columbus, Ohio, US; abstract no. 85111q, SIEVERS, R.E. ET AL. page 484 ; * abstract * & ADV. CHEM. SER. 1976, pages 222 - 231 | 1,4 | |

TECHNICAL FIELDS SEARCHED (Int. Cl.5 )

The present search report has been drawn up for all claims

| Place of search | Date of completion of the search | Examiner |
|---|---|---|
| THE HAGUE | 13 OCTOBER 1992 | RINKEL L.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)